# EUROPEAN PATENT APPLICATION

(11) **EP 2 361 625 A1**
(43) Date of publication of application: **31.08.2011**
(21) Application number: 10195408.9
(22) Date of filing: 19.09.2005
(51) Int. Cl.: A61K 31/4375, A61K 31/4725, A61P 3/06, A61P 9/10

(54) **Methods and compositions for the treatment of hyperlipidemia**

(30) Priority: 17.09.2004 CN 200410078150; 23.11.2004 CN 200410095066
(62) Divisional of application: 05791957.3
(71) Applicant: Institute of Medicinal Biotechnology, Chinese Academy of Medical Sciences, Beijing 100050 (CN)
(72) Inventor: Jiang, Jian-Dong, 100050, Beijing (CN); Kong, Wei-Jia, 100050, Beijing (CN); Zhao, Li-Xun, 100067, Beijing (CN); Song, Dan-Qing, 100078, Beijing (CN); Wei, Jing, 210006, Nanjing Beijing (CN)
(74) Representative: Walker, Ross Thomson

(57) **Abstract**

Methods and compositions containing a berberine compound or berberine related or derivative compound are provided for the prevention and treatment of hyperlipidemia, elevated cholesterol, and/or cardiovascular disease in mammalian subjects. The methods and compositions of the invention are effective for prevention and treatment of atherosclerosis, coronary artery disease, angina pectoris, carotid artery disease, stroke, cerebral arteriosclerosis, high blood pressure, myocardial infarction, cerebral infarction, restenosis following balloon angioplasty, intermittent claudication, dyslipidemia post-prandial lipidemia or xanthoma. Additional compositions and methods are provided which employ a berberine compound or berberine related or derivative compound in combination with a second anti-hyperlipidemia agent, or a different therapeutic agent to yield more effective treatment tools against hyperlipidemia and/or cardiovascular disease, and/or dual activity therapeutic methods and formulations useful to prevent or reduce hyperlipidemia and one or more causal or related symptoms or conditions associated with hyperlipidemia in mammalian subjects.

## Description

### Related Applications

This application claims all priority benefits of Chinese Patent Application No 200410095066 X, filed November 23, 2004, and of Chinese Patent Application No. 200410078150.0, filed September 17, 2004, each incorporated herein by reference.

### Technical Field

The present invention relates to methods and compositions for treating hyperlipidemia in mammalian subjects. More specifically, the invention relates to methods and compositions for treating and/or preventing hyperlipidemia as well as conditions or complications associated with hyperlipidemia in mammals.

### Background

Cardiovascular disease is currently the leading cause of death in the United States despite a 33% decrease in the incidence of the disease over the past 20 years. Several causative factors are associated with cardiovascular disease including infections, autoimmune response, hypercholesterolemia and hyperlipidemia. Approximately 90% of cardiovascular disease is diagnosed as atherosclerosis, which is caused by high blood plasma concentrations of low-density lipoproteins, (LDLs).

There are at least five distinct lipoproteins in mammals, each of which differs in size, composition, density and function. In the cells of the small intestine, dietary lipids are packaged into large lipoprotein complexes called "chylomicrons," which have a high triglyceride and low cholesterol content. In the liver, triglycerides and cholesterol esters are packaged and released into plasma as triglyceride-rich lipoproteins called very low-density lipoproteins (VLDLs), which primarily transport triglycerides made in the liver or released by adipose tissue. Through enzymatic action, VLDLs can either be reduced and taken up by the liver or transformed into intermediate density lipoproteins (IDLs). IDLs are in turn either taken up by the liver or further modified to form low density lipoproteins (LDLs). LDLs are either taken up and broken down by the liver, or taken up by extrahepatic tissue High density lipoproteins (HDLs) help remove cholesterol from peripheral tissues in a process called reverse cholesterol transport. Some forms of lipoproteins, such as LDLs, are considered "bad" cholesterol and increase the risk of heart disease or other diseases caused by hardening of the arteries. Other forms, such as HDLs, are considered "good" cholesterol and are essential for good health.

LDL metabolism is regulated by the liver low-density protein receptor (LDLR). Increased LDLR expression results in improved clearance of plasma LDL through receptor-mediated endocytosis, lowering plasma LDL levels and reducing the incidence of arterial plaque formation LDLR expression is generally regulated at the transcriptional level through a negative feedback mechanism by the intracellular cholesterol pool. This regulation is controlled through interactions of the sterol regulatory element (SRE-1) of the LDLR promoter and SRE binding proteins (SREBPs). In the inactive state, SREBP associates with SREBP-cleavage activating protein (SCAP). SCAP contains a cholesterol-sensing domain, which responds to the depletion of sterol with activation of the SCAP-SREBP transporting activity. Under cholesterol depleted conditions, SCAP transports SREBP to the Golgi apparatus where the N-terminal transcription activation domain for the SREBP is released from the precursor protein through specific cleavages. The active form of the SREBP translocates to the nucleus, binds to its cognate SRE-1 site and activates transcription of the LDLR gene. When there is enough cholesterol, the SCAP-SREBP complex remains in an inactive form in the endoplasmic reticulum through active repression by sterols, and LDLR gene transcription is maintained at a minimal constitutive level.

Deficiencies or failures of LDL regulatory mechanisms can result in hyperlipidemia, which is characterized by an abnormal increase in serum lipids in the bloodstream. Hyperlipidemia is a known causal factor for development of atherosclerosis and other cardiovascular and peripheral vascular diseases. Primary hyperlipidemia is generally caused by genetic defects, and secondary hyperlipidemia generally caused by secondary factors such as disease, drugs and/or dietary factors. Hyperlipidemia can also result from a combination of primary and secondary causes.

Primary hyperlipidemias include familial hyperchylomicronemia, familial hypercholesterolemia, familial combined hyperlipidemia, familial dysbetaliproteinemia, familial hypertriglyceridemia, and familial defective apolipoprotein B-100. Familial hyperchylomicronemia is a genetic disorder which results in a deficiency in an enzyme, LP lipase, that breaks down fat molecules. The LP lipase deficiency can cause the accumulation of large quantities of fat or lipoproteins in the blood. Familial hypercholesterolemia is caused by one or more mutations in the LDL receptor gene that result(s) in a malfunctioning LDL receptor or even complete absence of the LDL receptor. These genetic defects are associated with reduced or ineffective clearance of LDL, leading to elevated LDL and total cholesterol levels in the plasma. Familial combined hyperlipidemia, also known as multiple lipoprotein-type hyperlipidemia, can result in periodic elevation of cholesterol and triglyceride levels and a decrease in HDL levels. Familial defective apolipoprotein B-100 is an autosomal dominant genetic abnormality caused by a single nucleotide mutation that substitutes glutamine for arginine. This mutation leads to a reduced affinity of LDL particles for the LDL receptor, increasing plasma levels of LDL and total cholesterol. Familial dysbetaliproteinemia, also referred to as Type III hyperlipoproteinemia, results in moderate to severe elevations of serum triglyceride and cholesterol levels with abnormal apolipoprotein E function. In familial hypertriglyceridemia, the concentration of plasma VLDL is elevated. This can cause mild to moderately elevated triglyceride levels (and usually not elevated cholesterol levels) and can often be associated with low plasma HDL levels.

Secondary hyperlipidemia can be triggered by diseases such as uncontrolled diabetes mellitus (insulin-dependent diabetes mellitus and non-insulin-dependent diabetes mellitus) (Bianchi, R., et al., Diab. Nutr. Metabl. 7:43-51 (1994); Welborn, T.A., Aust. NZ J. Med. 24:61-54 (1994)), hypothyroidism, uremia, nephrotic syndrome, acromegaly, obstructive liver disease, and dysproteinemia (multiple myeloma, lupus erythematosus) (Harrison's Principles of Internal Medicine, Ed. Braunwald, E., et al., 11th Edition, McGraw-Hill 1016-1024 (1988)). A number of drugs can also produce secondary hyperlipidemia, including oral contraceptives, glucocorticoids and antihypertensives. Dietary factors such as increased caloric intake, recent weight gain, consumption of' foods high in saturated fats and cholesterol, and alcohol intake, can additionally contribute to the development of secondary hyperlipidemia.

Elevated lipoprotein levels, regardless of cause, are associated with a number of disease states, including atherosclerosis, coronary artery disease, angina pectoris, carotid artery disease, stroke, cerebral arteriosclerosis, myocardial infarction, cerebral infarction, restenosis following balloon angioplasty, high blood pressure, intermittent claudication, dyslipidemia, post-prandial lipidermia and xanthoma.

Lowering lipoprotein levels, including cholesterol levels, decreases the risks and/or severity of disease associated with hyperlipidemia Over the years many different strategies have been attempted to treat hyperlipidemia, with a principal focus on antidyslipidemic drugs--for which the global market in 2004 was estimated to be $26 billion (Commercial Insights Antiyslipidemics: Commercial Absorption of Lipitor's Dominance, Datamonitor, May, 2005). The most commonly prescribed drug treatments for hyperlipidemia and elevated cholesterol employ 3-hydroxy-3-methyl-glutaryl-CoA reductase (HMG-CoA reductase) inhibitors, also known as "statins". Stains lower cholesterol by slowing down the production of cholesterol and by increasing the liver's ability to clear LDL from the bloodstream. The newest statin is atorvastatin (Lipitor®) which is the only statin approved for the reduction of triglycerides as well as the reduction of total and LDL cholesterol. Other available statins which primarily reduce LDL cholesterols are cerivastatin (Baychol®), fluvastatin (Lescol®), lovastatin (Mevacor®), pravastatin (Pravachol®) and simvastatin (Zocor®). However, concerns regarding the incidence of liver failure and potentially fatal rhamhdomyolysis with the use of'statins have led to the development of adjunct therapies combining lower doses of statins with other medications. While this strategy has been somewhat effective, there remains an imporant need in the art for improved drug treatments to treat hyperlipidemia.

Another therapeutic approach attempted for lowering cholesterol employs bile acid sequestrants (e.g., cholestyramine, clofibrate), and/or nicotinic acid (niacin), which function by lowering elevated triglycerides However, bile acid sequestrants can cause gastrointenstinal problems such as constipation, abdominal pain, bloating, vomiting, diarrhea, weight loss, and flatulance, and can also decrease absorption of other medications. Nicotinic acid can cause liver problems, dizziness, and blurred vision, making these drugs unacceptable for many patients.

In addition to the use of conventional pharmaceutical therapies, traditional medicines (such as Chinese medicines and Japanese medicines) have been used for cardiovascular therapy for a number of years. These include the use of curcumin, also known as turmeric root, which is thought to reduce cholesterol in the body by blocking formation of thromboxane A2. Thromboxane A2 also functions to increase prostacyclin, a natural inhibitor of platelet aggregation, and thus also acts to inhibit blood clot formation (Srivastava et al., Arzneimittelforschung, 1986, 36(4): 715-17). Curcumin has been to decrease total cholesterol and LDL cholesterol levels in serum and to increase beneficial HDL cholesterol levels. Other herbal and botanical remedies that have been proposed for use in lowering cholesterol include gugulipid, made from the resin of Commiphora Mukul tree in India, garlic, vitamin E, soy, soluble fiber, carnitine, chromium coenzyme Q10, fiber, grape seed extract, pantothine, red yeast rice, royal jelly, fish oil, and green tea. The problem, however, is that most of these naturally-deiived, botanical and mineral products have consistent dosing problems, and many have unacceptable adverse side effects for at least some patients.

On average, 42% of the population among the top seven industrialized countries suffers from hyperlipidemia (Commercial Insights Antidyslipidemics: Commercial Absorption of Lipitor's Dominance, Datamonitor, May, 2005). In view of these statistics, and the current state of the art with respect to treating hyperlipidemia, there is a compelling, unmet need in the art to identify new compounds, formulations and methods to safely and effectively reduce plasma lipid levels in patients suffering from hyperlipidemia and other diseases and conditions associated with elevated lipid levels.

### Summary of the Exemplary Embodiments of the Invention

It is therefore an object of the present invention to provide novel and improved compositions and methods for treating and managing hyperlipidemia in mammalian subjects, including humans.

It is another object of the present invention to provide novel and improved compositions and methods for treating and managing cholesterol metabolic disorder in mammalian subjects, including humans.

It is a further object of the invention to provide compositions and methods for treating and preventing diseases triggered or aggravated by hyperlipidemia or elevated cholesterol including, but not limited to, cardiovascular diseases such as atherosclerosis, coronary artery disease, angina pectoris, carotid artery disease, stroke, cerebral arteriosclerosis, myocardial infarction, cerebral infarction, restenosis following balloon angioplasty, intermittent claudication, dyslipidemia post-prandial lipidemia, high blood pressure and xanthoma.

The invention achieves these objects and satisfies additional objects and advantages by providing novel and surprisingly effective methods and compositions for treating and/or preventing hyperlipidemia or elevated cholesterol in mammalian subjects employing berberine and related compounds and derivatives according to formula I, below. 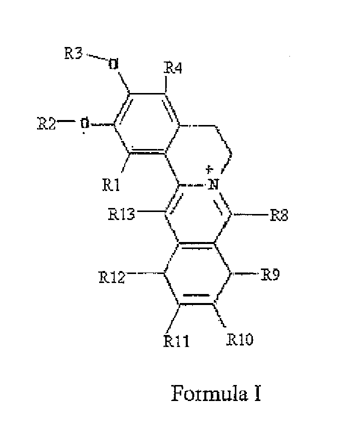 wherein each of R₁, R₂, R_{3,} R₄, R₈, R₉, R₁₀, R₁₁, R₁₂ and/or R₁₃ may independently, collectively, or in any combination that yields an active (e.g., anti-dyslipidemic, LDL-modulatoxy, or LDLR-modulatory) compound according to this disclosure, be a hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, or heterocyclo group. When more than one R group is present, the R group may be selected from any of'the stated groups so as to be the same or different. In certain exemplary embodiments, the following illustrative structural modifications according to Formula I above will be selected to provide useful candidate compounds for treating and/or preventing hyperlipidemia in mammalian subjects, e.g., wherein: R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl (e.g., substitution selected from methyl, ethyl, n-pnopyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-metlxyl-2ethylpropyl); R₉ is selected from methyl, ethyl, hydroxyl, Cl, Br; R₁₀ is selected from methyl, ethyl, hydroxyl, Cl, Br; R₁₁ is selected from methyl, ethyl, hydroxy, Cl, Br; R₁₂ is selected from methyl, ethyl, hydroxyl, Cl, Br; and R₁₃ is selected from straight or branched (C1-C6) alkyl (e.g., substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl). Additional candidate compounds for use within the compositions and methods will be readily produced and selected according to the further disclosure provided herein below.

Useful berberine and related compounds and derivatives within the formulations and methods of the invention include, but are not limited to, salts of berberine and related or derivative compounds, for example, berberine sulfate, berberine hydrochloride, berberine chloride, palmatine chloride, oxyberberine, dihydroberberine, 8-cyanodihydroberberine, tetrahydroberberine N-oxide, tetrahydroberberine, N-metlayltetrahydroberberinium iodide, 6 protoberberine, 9-ethoxycarbonyl berberine, 9-N,N-dimethylcarbamoyl berberine and 12-bromo berberine, berberine azide, and berberine betaine. Other seful forms of berberine and related compounds and derivatives for use within the invention include other pharmaceutically acceptable active salts of said compunds, as well as active isomers, enantiomers, polymorphs, solvates, hydrates, and/or prodrugs of said compounds.

In exemplary embodiments, the compositions and methods of the invention employ a berberine compound or a berberine related or derivative compound of Formula I to treat and/or prevent symptoms of hyperlipidemia or another disease or condition associated with hyperlipidemia, such as a cardiovascular disease.

Mammalian subjects amenable for treatment with berberine and berberine related and derivative compounds of Formula I according to the methods of the invention include, but are not limited to, subjects with hyperlipidemia and subjects with elevated cholesterol, including subjects presenting with, or at elevated risk for developing, elevated LDL, elevated cholesterol, and/or elevated triglyceride levels.

These and other subjects are effectively treated, prophylactically and/or therapeutically, by administering to the subject an-hyperlipidemia effective amount (or, alternatively, an anti-dyslipidemic, LDL-modulatory, or LDLR-modulatory effective amount) of a berberine or berberine related compound or derivative of Formula I sufficient to prevent or reduce hyperlipidemia or one or more disease symptoms or conditions associated with hyperlipidemia (or, alternatively, to elicit an anti-dyslipidemic, LDL-modulatory, or LDLR-modulatory response) in the subject). The therapeutically useful methods and formulations of the invention will effectively berberine and berberine related and derivative compounds of Formula I in a variety of forms, as noted above, including any active, pharmaceutically acceptable salt of said compounds, as well as active isomers, enantiomers, polymorphs, solvates, hydrates, prodrugs, and/or combinations thereof Berberine is therefore employed as an illustrative embodiment of the invention within the examples herein below.

In additional embodiments of the invention, mammalian subjects are effectively treated, prophylactically and/or therapeutically, by administering to the subject a cholesterol-controlling effective amount of a berberine compound or related or derivative compound of Formula I, sufficient to prevent or reduce elevated cholesterol, or one or more associated symptoms or condition(s), in the subject. These therapeutically useful methods and formulations of the invention may likewise employ a berberine compound or related or derivative compound of Formula I in a variety of forms, including pharmaceutically acceptable salts, isomers, enantiomers, polymorphs, solvates, hydrates, prodrugs, and/or combinations thereof.

Within additional aspects of the invention, combinatorial formulations and methods are provided which employ an effective amount of a berberine compound (or of another berberine related or derivative compound of formula I) in combination with one or more secondary or adjunctive active agent(s) that is/are combinatorially formulated or coordinately administered with the berberine or berberine related or derivative compound to yield an anti-hyperlipidemia or cholesterol lowering effective response (or, alternatively, an anti-dyslipidemic, LDL-moduiatory, or LDLR-modulatory response) in the subject. Exemplary combinatorial formulations and coordinate treatment methods in this context employ the berberine or berberine related or derivative compound of Formula I in combination with one or more additional, lipid lowering agent(s) or other indicated, secondary or adjunctive therapeutic agents. The secondary or adjunctive therapeutic agents used in combination with, e.g., berberine in these embodiments may possess direct or indirect lipid lowering activity, including cholesterol lowering activity, alone or in combination with, e.g., berberine, or may exhibit other useful adjunctive therapeutic activity in combination with, e.g., berberine. Useful adjunctive therapeutic agents in these combinatorial formulations and coordinate treatment methods include, for example, antihyperlipidemic agents; antidyslipidemic agents; plasma HDL-raising agents; antihypercholesterolemic agents, including, but not limited to, cholesterol-uptake inhibitors; cholesterol biosynthesis inhibitors, e.g., HMG-CoA reductase inhibitors (also referred to as statins, such as lovastatin, simvastatin, pravastatin, fluvastatin, rosuvastatin, pitavastatin, and atorvastatin); HMG-CoA synthase inhibitors; squalene epoxidase inhibitors or squalene synthetase inhibitors (also known as squalene synthase inhibitors); acyl-coenzyme. A cholesterol acyltransferase (ACAT) inhibitors, including, but not limited to, melinamide; probucol; nicotinic acid and the salts thereof; niacinamide; cholesterol absorption inhibitors, including, but not limited to, β-sitosterol or ezetimibe; bile acid sequestrant anion exchange resin, including, but not limited to cholestyramine, colestipol, colesevelam or dialkylaminoalkyl derivatives of a cross-linked dextran; LDL receptor inducers; fibrates, including, but not limited to, clofibrate, bezafibrate, fenofibrate and gemfibrozil; vitamin B6 (also known as pyridoxine) and the pharmaceutically acceptable salts thereof, such as the HCl salt; vitamin B12 (also known as cyanocobalamin); vitamin B3 (also known as nicotinic acid and niacinamide, supra); anti-oxidant vitamins, including, but not limited to, vitamin C and E and betacarotene; β blockers; angiotensin II receptor (AT₁) antagonist; angiotensin-converting enzyme inhibitors, renin inhibitors; platelet aggregation inhibitors, including, but not limited to, fibrinogen receptor antagonists, i.e., glycoprotein IIb/IIIa fibrinogen receptor antagonists; hormones, including but not limited to, estrogen; insulin; ion exchange resins; omega-3 oils; benfluorex; ethyl icosapentate; and amlodipine. Adjunctive therapies may also include increases in exercise, surgery, and changes in diet (e.g., to a low cholesterol diet). Some herbal remedies may also be empoyed effectively in combinatorial formulations and coordinate therapies for treating hyperlipidemia, for example curcumin, gugulipid, garlic, vitamin E, soy, soluble fiber, fish oil, green tea, carnitine, chromium, coenzyme Q10, anti-oxidant vitamins, grape seed extract, pantothine, red yeast rice, and royal jelly.

The forgoing objects and additional objects, features, aspects and advantages of the instant invention will become apparent from the following detailed description.

### Brief Description of the Drawings

Figure 1 is a drawing of the promoter region of the LDL receptor gene. Three direct repeats and two TATA-like sequences are identified with the promoter region The cis-acting element of sterols is located on repeat 2, whereas the regulatory element for cytokine OM (SIRE) overlaps the TATA-like sequences.

Figure 2 is a schematic representation of intracellular regulation of LDL receptor gene expression, including regulation by berberine.

Figures 3 A and B are quantitative RI-PCR of LDLR mRNA levels in human liver BEL-7402 cells twenty-four hours after being treated with berberine (A) or berberine sulfate (B).

Figure 4 is a measurement using flow cytometry of the concentration of the protein level of LDLR expressed on the cell surface of BEL-7402 cells twenty-four hours after treatment with 15µg/ml of berberine.

Figures 5 A-C are charts of the decrease in serum cholesterol (A) and LDL (B) in hamsters after treatment with berberine and the decrease of LDL as a function of time (C).

Figure 6 is a depiction of the concentration of'total LDLR mRNA and protein extracts as measured by quantitative real time RT-PCR (A) and Western blot (B) in hamsters sacrificed four hours after the last treatment with berberine.

Figure 7 is a Western Blot showing the concentration of the precursor (P) and mature (M) forms of SREBP2 using a monoclonal antibody to SREBP2 in HepG2 cells.

Figure 8 is (A) a northern blot showing LDLR expression in HepG2 cells treated with either lovastatin (Lov) alone or in combination with berberine (BBR) for 24 hours and (B) a chart of real-time RT-PCR of'the same cells.

Figure 9 is a chart showing the increase in LDLR promoter activity in the presence of GW707 and oncostatin M.

Figure 10 is (A) a northern blot showing concentrations of LDLR mRNA in HepG2 cells treated with berberine in the presence of different concentrations of actinomycin D and (B) a plot of normalized LDLR mRNA signals as a percentage of LDLR mRNA remaining.

Figure 11 is a schematic representation of the *LDLR* mRNA 3' UTR and the chimeric Luc-LDLR 3' UTR constructs.

Figure 12 is a northern blot of analysis of Luc-LDLR fusion mRNA in (A) control cells and cells treated with (B) berberine or dimethylsulfoxide as a control.

Figure 13 is a schematic representation of the constructs containing the deletions of ARE and UCAU motifs (B) and a chart illustrating the responses of the wt pLuc/UTR-2 and deletion constructs to berberine treatment as determined by real-time RT-PCR analysis,

Figure 14 is a western blot of cellular proteins harvested from (A) Bel-7402 cells or (B) HepG2 cells that were untreated or treated with berberine at a dose of 5µg/ml for different levels as indicated and (C) a western blot of HepG2 cells treated for 1 hour at the indicated concentrations.

### Detailed Description of Exemplary Embodiments of the Invention

The instant invention provides novel methods and compositions for preventing and/or treating hyperlipidemia and elevated cholesterol in mammalian subjects, including individuals and *in vitro, ex vivo,* and *in vivo* mammalian cells, tissues, and organs. In various embodiments, the methods and compositions are effective to prevent or treat diseases caused by hyperlipidemia and elevated cholesterol, including cardiovascular disease. As used herein, the term "cardiovascular disease" is intended to include a range of symtoms, conditions, and/or diseases including atherosclerosis, coronary artery disease, angina pectoris, carotid artery disease, stokes, cerebral arteriosclerosis, myocardial infarction, high blood pressure, cerebral infarction, restenosis following balloon angioplasty, intermittent claudication, dyslipidemia post-prandial lipidemia and xanthoma, and all conventionally targeted symptoms arising from or associated with the foregoing diseases and conditions.

Lipid lowering formulations and methods provided herein, including cholesterol lowering formulations and methods, employ a berberine compound or berberine related or derivative compound of Formula I, above, including all active pharmaceutically acceptable compounds of this description as well as various foreseen and readily provided complexes, derivatives, salts, solvates, isomers, enantiomers, polymorphs, and prodrugs of these compounds, and combinations thereof, as novel lipid lowering agents. Exemplary compounds for use within the invention include, as illustrative embodiments, berberine sulfate, berberine chloride, berberine hydrochloride, palmatine chloride, oxyberberine, dihydroberberine, 8-cyanodihydroberberine, tetrahydroberberine N-oxide, tetrahydroberberine, N-methyltetrahydroberiberinium iodide, 6-protoberberine, 9-ethoxycaxbonyl berberine, 9-N,N-dimethylcarbamoyl berberine and 12-bromo berberine, berberine azide, and berberine betaine.

Within the formulations and methods, a berberine compound or berberine related or derivative compound as disclosed herein is effectively used to treat hyperlipidemia and elevated cholesterol levels in mammalian subjects suffering from hyperlipidemia and/or elevated cholesterol and conditions associated with hyperlipidemia and elevated cholesterol including cardiovascular diseases such as, but not limited to, atherosclerosis, coronary artery disease, angina pectoris, carotid artery disease, strokes, cerebral arteriosclerosis, myocardial infarction, cerebral infarction, restenosis following balloon angioplasty, high blood pressure, intermittent claudication, dyslipidemia post-prandial lipidemia and xanthoma.

A broad range of mammalian subjects, including human subjects, are amenable to treatment using the formulations and methods of the invention. These subjects include, but are not limited to, human and other mammalian subjects presenting with hyperlipidemia or elevated cholesterol levels or diseases aggravated or triggered by hyperlipidemia such as cardiovascular diseases, including, atherosclerosis, coronary artery disease, angina pectoris, carotid artery disease, strokes, cerebral arteriosclerosis, myocardial infarction, cerebral infarction, restenosis following balloon angioplasty, intermittent claudication, high blood pressure, dyslipidemia post-prandial lipidemia and xanthoma.

Within the methods and compositions of the invention, one or more berberine compound(s) or berberine related or derivative compound(s) as disclosed herein is/are effectively formulated or administered as an anti-hyperlipidemia or cholesterol lowering agent effective for treating hyperlipidemia and/or related disorders in exemplary embodiments, berberine chloride is demonstrated for illustrative purposes to be an anti-hyperlipidemia effective agent in pharmaceutical formulations and therapeutic methods, alone or in combination with one or more adjunctive therapeutic agent(s). The present disclosure further provides additional, pharmaceutically acceptable berberine compounds and berberine related and derivative compounds in the form of a native or synthetic compound, including complexes, derivatives, salts, solvates, isomer, enantiomers, polymorphs, and prodrugs of the compounds disclosed herein, and combinations thereof, which are effective as lipid lowering therapeutic agents within the methods and compositions of the invention.

Hyperlipidemia is an abnormal increase in serum lipids in the bloodstream. It is generally classified as primary hyperlipidemia, which is caused by genetic defects; or secondary hyperlipidemia, which is caused by various disease states, drugs and/or dietary factors, Hyperlipidemia may also result from a combination of primary and secondary causes of hyperlipidemia. The compositions and methods of'the present invention are effective in the treatment of all types of hyperlipidemia, regardless of cause.

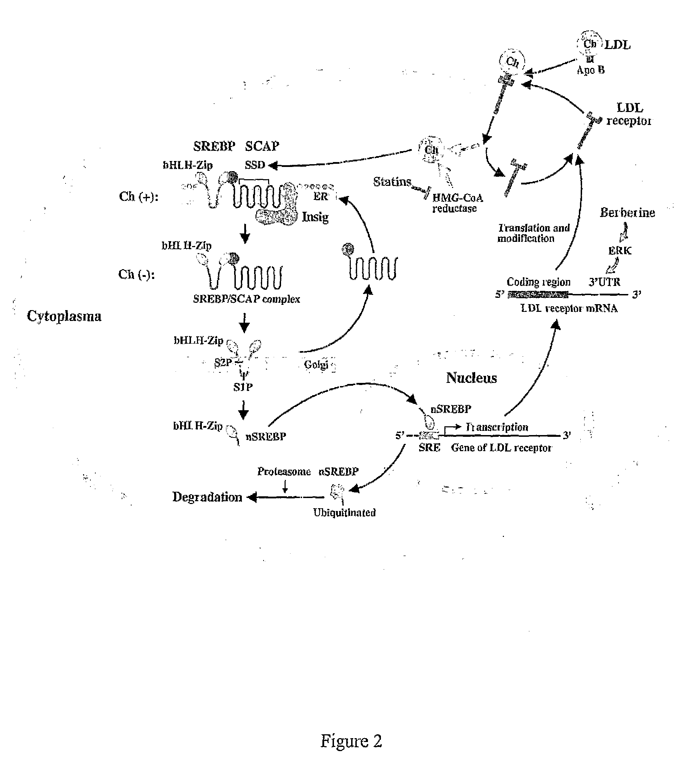

One cause of hyperlipidemia and elevated cholesterol levels is the failure of one or more LDL regulatory mechanisms or pathways. LDL concentrations in plasma ate regulated in part by the LDL receptor which captures LDL particles from the bloodstream and draws them inside the cell, clearing them from the bloodstream when there is too much and releasing them when more LDL is needed. Transcriptional regulation of the LDL receptor gene is controlled through the sterol regulatory element-binding protein pathway(SREBP). Bile acid sequestrants, cholesterol biosynthesis inhibitors, and cholesterol absorption inhibitors all influence the SREBP pathway and subsequently upregulate LDL receptor expression. The statins competitively inhibit 3-hydroxy-3-methyl-glutatyl-CoA reductase (HMG-CoA reductase) and block cholesterol biosynthesis in the liver. Hormones, cytokines, growth factors and second messengers also regulate transcription of the LDL receptor gene as outlined in Table 1, below. Post-transcriptional control of the LDL receptor gene is also a target for pharmaceutical intervention It has been determined in the present invention that berberine is capable of upregulating LDL receptor expression through a post-transcriptional and sterol independent mechanism in hepatocytes (Fig. 2).

**Table 1 Upregulation of LDL receptor gene expression by different agents**

| Agent(s) | Sites of action | Sterol-dependence | *Cis-acting element(s)* | Trans*-acting Factors* | *Signaling* pathway |
|---|---|---|---|---|---|
| Statins | Transcription | Dependent | SRE | SREBPs (activated by proteolytic cleavage) | |
| Estrogens | Transcription | Independent | Repeat 3 | Estrogen receptot-α and Sp1 | |
| Insulin/ growth factors | Transcription | Independent | SRE/SRE + repeat 1 and 3 | SREBPs (activated by phosphorylation)/SR EBPs + Sp1 | ERK* |
| INF-α/ IL-1] | Tanscription | Dependent | Unidentified | Unidentified | ERK |
| OM | Transcription | Independent | SIRE | Egr 1 and c/EBP β | ERK |
| PMA | Transcription/ post-transcription | Independent | Unidentified/ 3' sequence of LDL receptor 3'UTR | Unidentified | PKC |
| Berberine | Post-transcription | Independent | 5' sequence of LDL receptor 3' UTR | Unidentified | ERK |

| | | | | | |
|---|---|---|---|---|---|
| *c/EBP: CCAAT/enhancer binding protein; Egr1: early growth response gene 1; ERK: extracellular signal-regulated kinase; IL: interleukin; LDL: low density lipoprotein; OM: oncostatin M; PKC: protein kinase C; PMA: phorbol-12-myristate-13-acetate; SIRE: sterol-independent regulatory element; SRE: sterol regulatory element; SREBP: sterol regulatory element-binding protein; TNF: tumor necrosis factor; UTR: untranslated region. | | | | | |

Those skilled in the art will appreciate that each of the forgoing agents identified in Table 1 that possess activity for regulating LDL receptor expression are useful in combination with the berberine compounds and berberine related and derivative compounds described herein, within various combinatorial formulations and coordinate administration methods as described in detail below

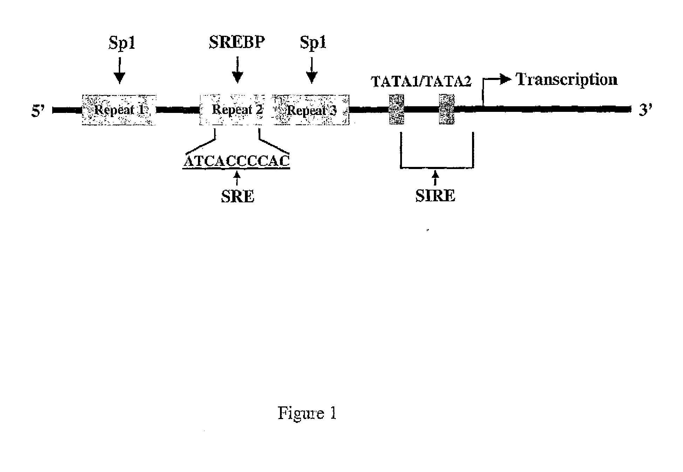

The human LDL receptor structural gene is located in the short arm of chromosome 19. It spans approximately 45 kilobases (kb) and consists of 18 exons, each coding for a different protein domain and 17 introns. (Lindgren et al., PNAS 82:8567-8571 (1985)). The promoter is located on the 5'-flanking region, within which the majority of *cis*-acting DNA elements are found between base pair (bp) -58 and -234, with the A of'the initiator methionine codon as +1. The promoter region spans 177 bp, including three imperfect direct repeats with 16 bp of each, two TATA-like sequences, and several transcription initiation sites, all of which are essential for gene expression and regulation (Fig 1) (Südhoff et al, Science 228:815-822 (1987)) Repeat 2 contains the 10 bp DNA sterol regulatory element (SRE) (Fig. 1, Smith et al, J. Biol Chem 265:2306-2310 (1990)) which controls transcription of the LDL regulator. The human LDL receptor mRNA has a 5.3 kb sequence in length, which contains an unusually 25 kb long 3' untranslated region (UTR) (Yamamoto, Cell 39:27-38 (1984)). There are three AU rich elements (AREs) in the 5' proximal region and three copies of *Alu*-like repeat in the 3' distal region of the 3'UTR. These structures play a key role in the stability of the LDL receptor mRNA which has a constitutively short half life of about 45 minutes in HepG2 cells, and serve as cis-acting elements for the post-transcriptional regulation of the LDL receptor gene expression (Yamamoto et al., Cell 39:27-38 (1984) and Wilson et al., J. Lipid Res. 39:1025-1032 (1998)).

The sterol regulatory element-binding proteins (SREBP) are transcription factors belonging to the basic-helix-loop-helix-leucine zipper (bHLH-Zip) family (Yokoyama et al, Cell 75:187-197 (1993)). They bind to sterol regulatory element (SRE), which is not only present in the promoter of the LDL receptor gene but also in promoters of other genes that code for enzymes participating in cholesterol or fatty acid biosynthesis, such as the HMG-CoA reductase gene and the acetyl coenzyme A synthetase gene (Rawson et al, Mol, Cell. Biol 4:631-640 (2003)). The major activator of the LDL receptor gene is SREBP-2 (Horton, et al, J. Clin. Invest. 109:1125-1131 (2002).

When cholesterol or its derivatives are abundant in cells, the SREBP pathway is suppressed and the transcription of the LDL receptor gene or other genes required for lipid synthesis are turned off Abundant cholesterol binds directly to the sterol sensing domain (SSD) of the SREB cleavage-activating protein (SCAP) causing a conformational change which permits SCAP to bind to a pair of endoplasmic reticulum membrane proteins named insulin-induced genes (Insig) 1 and 2, then forms SREBP/SCAP/Insig ternary complex (Fig 2) (Yang et al., Cell 110:489-500 (2002)) This traps SREBP/SCAP in the endoplasmic reticulum membrane so that the SREBPs are not able to get to the Golgi apparatus for cleavage and the expression levels of LDLR decreases accordingly As a result, the uptake and synthesis of cholesterol are inhibited, and the cells reach a cholesterol homeostasis (Yang et al., Cell 110:4489-500 (2002)).

When sterols are absent, SCAP does not interact with the Insig proteins. Instead, the SREBP/SCAP complex is free to leave the endoplasmic reticulum and enter the Golgi apparatus (Espenshade et al., PNAS 99:11694-11699 (2002) After arriving in the Golgi apparatus, the transcriptional active domain of the SREBP precursor is released by two sequential proteolytic cleavage catalyzed by two proteases residing in the Golgi membrane, while SCAP returns to the endoplasmic reticulum (Fig. 2) (Brown et al, PNAS 96:11041-11048 (1999) and Nohtturfft et al., PNAS 96:11235-11240 (1999). The cleavage of the SREBP precursor results in the release of a fragment containing the bHLH-Zip domain; termed nuclear SREBP (nSREBP), or the mature form of SREBP The nSREBP enters into the nucleus and activates the transcription LDLR (Brown et al., PNAS 96:11041-11048 (1999). As a result, the cells uptake more cholesterol-containing lipoproteins and increase cholesterol production to reach a new level of cholesterol homeostasis. The nSREBP is not stable, and is polyubiquitinated and rapidly degraded by the proteasome with an estimated half-life of 3 hours (Hirano, et al., J. Biol Chem. 276:36431-36437 (2001)).

LDL receptor expression can be regulated by such factors as hormones, including estrogen which as an atheroprotective effect and triiodothyronine; insulin and several cytokines including tumor necrosis factor (TNF) α, Interleukin (IL) 1, IL-6 and oncostatin M (OM) all of which activate the transcription of the LDL receptor gene in hepatocytes (Stopeck et al, J. Biol Chem. 268:17489-17494 (1993)) (Table 1) INF-α and IL-1 are capable of regulating the LDL receptor gene transcription only when cells are cultured in sterol-free media, and their induction is repressed after sterols or LDL is added (Stopeck et al., J Biol Chem 268:17489-17494 (1993)). OM or IL-6 upregulate the LDL receptor gene expression in a sterol-independent manner, similar to that of insulin and some growth factors (Gierens et al., Arterioscler. Thromb. Vasc. Biol. 20:1777-1783 (2000)). OM has also been shown to increase the LDL receptor gene transcription by recruiting transcription factors early growth response gene 1 (Egr1) and CCAAI/enhancer binding protein β (c/EBP β) to bind to a DNA motif termed sterol-independent regulatory element (SIRE) which overlaps the TAIA-like sequences in the promoter region of' the LDL receptor gene (Fig 1), whereas IL-6 needs SRE and the repeat 3 Sp1 binding site for mediating its transcriptional activation effect factors (Gierens et al, Arterioscler Thromb. Vasc. Biol. 20:1777-1783 (2000)).

Growth factors including the platelet-derived growth factor (PDGF), EGF and the fibroblast growth factor (FGF) also upregulate LDL receptor gene expression (Basheeruddin et al., Arterioscler. Thromb. Vasc. Biol 15:1248-1254 (1995)). The stimulation effect of growth factors on the LDL receptor gene promoter requires SRE as well as the Sp1 binding sites as *cis-*acting elements, and is related to the ERK mediated phosphorylation and activation of SREBPs, as growth factors potently activate this signaling pathway just like insulin (Kotzka et al., J. Lipid. Res 41:99-108 (2000)). Second messenger analog phorbol esters regulate the LDL receptor gene expression as well.

The above-mentioned extracellular stimuli appear to require the activation of the ERK signaling cascade. Blocking the ERK pathway stops their ability to regulate LDL receptor gene expression (Kumar et al., J Biol Chem 275:5214-4221 (1998). ERK belongs to the subfamilies of the mitogen-activated protein kinases (MAPK), the activation of which by successive phosphorylation is secondary to the extracellular stimuli binding to their receptors on cell surface. These receptors either have intrinsic tyrosine kinase activity (like growth factor receptors and insulin receptor) or are coupled to another protein-tyrosine kinase (like receptor for cytokines) (Robinson, Curr Opin. Cell Biol 9:180-186 (1997). Upon activation, ERK phosphorylates and activates numerous cytoplasmic or nuclear protein factor, and mediates multiple biological responses including those that control cell growth and differentiation But how the ERK pathway links to the promoter of the LDL receptor gene and increases its transcription through different mechanisms has not been previously elucidated In the present invention, as described in Example XI below, it was determined that berberine rapidly activates ERK and that the kinetics of ERK activation preceded the upregulation of LDLR expression by berberine, ERK activation was also determined to be important in berberine's stabilization of LDLR mRNA.

As shown in the examples below, berberine and its analogs exercise post-transcriptional control of the LDL receptor as illustrated in Figure 2 Berberine is a quaternary alkaloid widely distributed in nine plant families of the structure of the compound of formula II

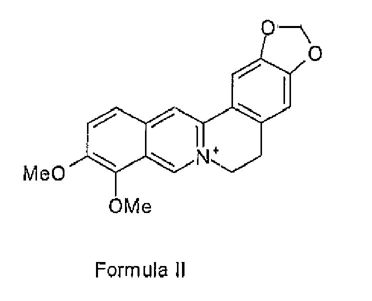

Berberine can be found in *Hydrastis canadensis* (goldenseal), *Coptis chinensis* (Coptis or goldenthread), *Berberis aquifolium* (Oregon grape), *Berberis vulgaris* (barberry), *Berberis aristata* (tree turmeric), *Chinese Isatis, Mahonia swaseyi, Yerba mansa* (Anemopsis californica) and *Phellodendron amurense* Products from these and other berberine-containing herbal sources, including any preparation or extract therefrom, are contemplated as useful compositions comprising berberine (or berberine analogs, related compounds and/or derivatives) for use within the invention. Useful berberine compounds and berberine related and derivative compounds for use within the invention will typically have a structure as illustrated in Formula I, although functionally equivalent analogs, complexes, conjugates, and derivatives of such compounds will also be appreciated by those skilled in the art as within the scope of at least certain aspects of this invention.

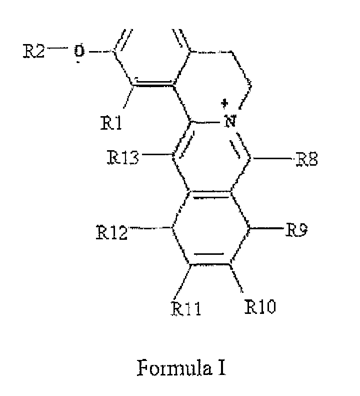

Useful berberine compounds and berberine related and derivative compounds for use within the invention according to Formula I will also typically have a structure wherein R₁, R₂. R₃, R₄ R₈, R₉, R_{10,} R₁₁, R₁₂ and/or R₁₃ is selected (each independently, and in any combination yielding an active comound as described) from a halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, or heterocyclo group.

In more detailed embodiments, illustrative structural modifications according to Formula I above will be selected to provide useful candidate compounds for treating and/or preventing hyperlipidemia in mammalian subjects wherein: R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl (e.g, substitution selected from methyl, ethyl, n-propyl, 1-methylethyl, n-butyl, 1-methylpropyl, 2 methylpropyl, 1,1-dimethylethyl, n-pentyl, 2-methylbutyl, 1,1-dimethylpropyl, 2,2 dimethylpropyl, .3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl); R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxy, C1, Br; and R₁₃ is selected from straight or branched (C1-C6) alkyl (e.g, substitution selected from methyl, ethyl, n-propyl, 1 -methylethyl, n-butyl, 1-methylpropyl, 2-methylpropyl, 1,1- dimethylethyl, n-pentyl, 2-methylbutyl, 1,1-dimethylpropyl, 2,2 dimethylpropyl, 3-methylbutyl, n-hexyl, 1,-methylpentyl, 1,1-dimethylbutyl, 2,2-dimethylbutyl, 3-methylpentyl, 1,2-dimethylbutyl, 1,3-dimethyl and 1-methyl-2ethylpropyl) Yet additional candidate compounds for use within the compositions and methods of the invention are provided wherein each of the R₁, R_{2,} R₃, R₄ R₈, R₉,R₁₀, R₁₁, and/or R₁₂ groups indicated in Formula I can be optionally (independently, collectively, or in any combination yielding an active compound as described) substituted as described and defined in the following passages.

The term "halogen" as used herein refers to bromine, chlorine, fluorine or iodine. In one embodiment, the halogen is fluorine. In another embodiment, R₉, R₁₀, R₁₁, R₁₂ and/or R₁₃ may independently be chlorine or bromine

The term "hydroxy" as used herein refers to -OH or -O⁻.

The term "alkene" as used herein refers to unsaturated hydrocarbons that contain carbon-carbon double bonds Examples of such alkene groups include ethylene, propene, and the like. In one embodiment, R₂ and/or R₃ may independently be methene.

The term "alkyl" as used herein refers to straight- or branched-chain aliphatic groups containing 1-20 carbon atoms, preferably 1-7 carbon atoms and most preferably 1-6 carbon atoms. This definition applies as well to the alkyl portion of alkoxy, alkanoyl and aralkyl groups. In one embodiment, R₁ R₂ R₃, R₄, R₈ and/or R₁₃ may independently be methyl or ethyl groups. In another embodiment R₈ and/or R₁₃ may independently be n-propyl, 1-methylethyl, n-butyl, 1-methylpropyl, 2-methylpropyl, 1,1 dimethyleledhyl, n-pentyl, 2-methylbutyl, 1,1-dimethylpropyl, 2,2-dimethylpropyl, 3-methylbutyl, m-hexyl, 1-methylpentyl, 1,1-dimethylbutyl, 2,2-dimethylbutyl, 3-methylpentyl, 1-2-dimethylbutyl, 1,3-dimethyl or 1-methyl-2ethylpropyl.

The term "alkoxy" includes substituted and unsubstituted alkyl, alkenyl, and alkynyl groups covalently linked to an oxygen atom. In one embodiment, the alkoxy group contains 1 to 6 carbon atoms Embodiments of alkoxy groups include, but are not limited to, methoxy, ethoxy, isopropyloxy, propoxy, butoxy, and pentoxy groups. In a further embodiment, R₉, R₁₀, R₁₁ and/or R₁₂ may independently be methoxy or ethoxy groups. In another embodiment, R₁ is a methoxy group. Embodiments of substituted alkoxy groups include halogenated alkoxy groups. In a further embodiment, the alkoxy groups can be substituted with groups such as alkenyl, alkenyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkylamino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Exemplary halogen substituted alkoxy groups include, but are not limited to, fluoromethoxy, difluoromethoxy, trifluoromethoxy, chloromethoxy, dichloromethoxy, and trichloromethoxy. In one embodiment, R₁, R₄, R₉, R₁₀, R₁₁ and/or R₁₂ may independently be an hydroxyl group.

The term "nitro", as used wherein alone or in combination refers to a -NO₂ group.

The term "amino" as used herein refers to the group --NRR', where R and R' may independently be hydrogen, alkyl, aryl, alkoxy, or heteroaryl. The term "aminoalkyl" as used herein represents a more detailed selection as compared to "amino" and refers to the group-NRR', where R and R' may independently be hydrogen or (C₁-C4)alkyl.

The term "trifluoromethyl" as used herein refers to -CF₃.

The tenn "trifluoromethoxy" as used herein refers to --OCF₃.

The term "cycloalkyl" as used herein refers to a saturated cyclic hydrocarbon ring system containing from 3 to 7 carbon atoms that may be optionally substituted. Exemplary embodiments include, but are not limited to, cyclopropyl, cyclobutyl, cyclopentyl and cyclohexyl. In certain embodiments, the cycloalkyl group is cyclopropyl. In another embodiment, the (cycloalkyl)alkyl groups contain from 3 to 7 carbon atoms in the cyclic portion and 1 to 4 carbon atoms in the alkyl portion. In certain embodiments, the (cycloalkyl)alkyl group is cyclopropylmethyl. The alkyl groups are optionally substituted with from one to three substituents selected from the group consisting of halogen, hydroxy and amino.

The terms "alkanoyl" and "alkanoyloxy" as used herein refer, respectively, to -C(O)-alkyl groups and -O-C(O)-alkyl groups, each optionally containing 2-5 carbon atoms. Specific embodiments of alkanoyl and alkanoyloxy groups are acetyl and acetoxy, respectively.

The term "aryl" as used herein refers to monocyclic or bicyclic aromatic hydrocarbon groups having from 6 to 12 carbon atoms in the ring portion, for example, phenyl, naphthyl, biphenyl and diphenyl groups, each of which maybe substituted with, for example, one to four substituents such as alkyl; substituted alkyl as defined above, halogen, trifluoromethyl, trifluoromethoxy, hydroxy, alkoxy, cycloalkyloxy, alkanoyl, alkanoyloxy, amino, alkylamino, dialkylamino, nitro, cyano, carboxy, carboxyalkyl, carbamyl, carbamoyl and aryloxy. Specific embodiments of aryl groups in accordance with the present invention include phenyl, substituted phenyl, naphthyl, biphenyl, and diphenyl.

The term "aroyl," as used alone or in combination herein, refers to an aryl radical derived from an aromatic carboxylic acid, such as optionally substituted benzoic of naphthoic acids.

The term "nitrile" or "cyano" as used herein refers to the group -CN.

The term "dialkylamino" refers to an amino group having two attached alkyl groups that can be the same or different.

The term "alkenyl" refers to a straight or branched alkenyl group of 2 to 10 carbon atoms having 1 to 3 double bonds. Preferred embodiments include ethenyl, 1-propenyl, 2-propenyl, 1-methylethenyl, 1-butenyl, 2-butenyl, 3-butenyl, 2-methyl-2-propenyl, 1-pentenyl, 2-pentenyl, 4-pentenyl, 3-methyl-2-butenyl, 1-hexenyl, 2-hexenyl, 1-heptenyl, 2-heptenyl, 1-octenyl, 2-octenyl, 1,3-octadienyl, 2-nonenyl, 1,3-nonadienyl, 2-decenyl, etc.

The term "alkynyl" as used herein refers to a straight or branched alkynyl group of 2 to 10 carbon atoms having 1 to 3 triple bonds Exemplary alkynyls include, but are not limited to, ethynyl, 1-propynyl, 2-propynyl, 1-butynyl, 2-butynyl, 3-butynyl, 1-pentynyl, 2-pentynyl, 4-pentynyl, 1-octynyl, 6-methyl-1-heptynyl, and 2-decynyl.

The term "hydroxyalkyl" alone or in combination, refers to an alkyl group as previously defined, wherein one or several hydrogen atoms, preferably one hydrogen atom has been replaced by a hydroxyl group. Examples include hydroxymethyl, hydroxyethyl and 2-hydroxyethyl.

The term "aminoalkyl" as used herein refers to the group --NRR', where R and R' may independently be hydrogen or (C₁-C₆)alkyl.

The term "alkylaminoalkyl" refers to an alkylamino group linked via an alkyl, group (i.e, a group having the general structure --alkyl-NH-alkyl or --alkyl-N(alkyl)(alkyl)). Such groups include, but are not limited to, mono- and di-(C₁-C₈ alkyl)aminoC₁ -C₈ alkyl, in which each alkyl may be the same or different.

The term "dialkylaminoalkyl" refers to alkylamino groups attached to an alkyl group. Examples include, but are not limited to, N,N-dimethylaminomethyl, N,N-dimethylaminoethyl N,N-dimethylaminopropyl, and the like The term dialkylaminoalkyl also includes groups where the bridging alkyl moiety is optionally substituted.

The term "haloalkyl" refers to an alkyl group substituted with one or more halo groups, for example chloromethyl, 2-bromoethyl, 3-iodopropyl, trifluoromethyl, perfluoropropyl, 8-chlorononyl and the like.

The term "carboxyalkyl" as used herein refers to the substituent -R'-COOH wherein R' is alkylene; and carbalkoxyalkyl refers to -R'-COOR wherein R' and R are alkylene and alkyl respectively In certain embodiments, alkyl refers to a saturated straight- or branched-chain hydrocarbyl radical of 1-6 carbon atoms such as methyl, ethyl, n-propyl, isopropyl, n-butyl, t-butyl, n-pentyl, 2-methylpentyl, n-hexyl, and so forth. Alkylene is the same as alkyl except that the group is divalent.

The term "alkoxyalkyl" refers to a alkylene group substituted with an alkoxy group For example, methoxyethyl [CH₃OCH₂CH₂-] and ethoxymethyl (CH₃CH₂OCH₂-] are both C₃ alkoxyalkyl groups.

The term "carboxy", as used herein, represents a group of the Formula -COOH.

The term "alkanoylamino" refers to alkyl, alkenyl or alkynyl groups containing the group --C(O)-- followed by --N(H)--, for example acetylamino, propanoylamino and butanoylamino and the like.

The term "carbonylamino" refers to the group NR--CO--CH₂--R', where R and R' may be independently selected from hydrogen or (C₁-C₄)alkyl.

The term "carbamoyl" as used herein refers to --O--C(O)NH₂.

The term "carbamyl" as used herein refers to a functional group in which a nitrogen atom is directly bonded to a carbonyl, i e , as in --NRC(=O)R' or --C(=O)NRR', wherein R and R' can be hydrogen, alkyl, substituted alkyl, alkenyl, substituted alkenyl, alkoxy, cycloalkyl, aryl, heterocyclo, or heteroaryl.

The term "alkylsulfonylamino" refers to refers to the group --NHS(O)₂Rₐ wherein Rₐ is an alkyl as defined above.

The term "heterocyclo" refers to an optionally substituted, unsaturated, partially saturated, or fully saturated, aromatic or nonaromatic cyclic group that is a 4 to 7 membered monocyclic, or 7 to 11 membered bicyclic ring system that has at least one heteroatom in at least one carbon atom containing ring. The substituents on the heterocyclo rings may be selected from those given above for the aryl groups. Each ring of the heterocyclo group containing a heteroatom may have 1, 2 or 3 heteroatoms selected from nitrogen atoms, oxygen atoms and sulfur atoms. Plural heteroatoms in a given heterocyclo ring may be the same or different.

All value ranges expressed herein, are inclusive over the indicated range. Thus, a range of R between 0 to 4 will be understood to include the values of 1,2, 3, and 4.

Berberine chloride is one exemplary form of berberine for use within the methods and compositions of the invention, having the structure of formula III below

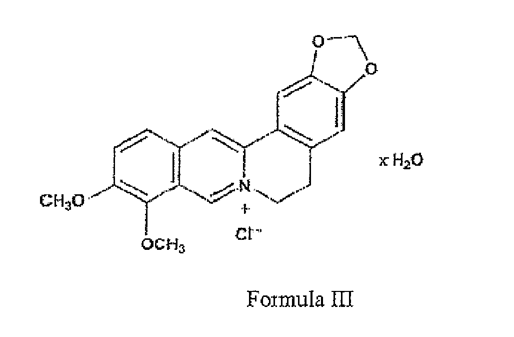

While berberine and related and derivative compounds maybe generated by any methods known to those skilled in the art, exemplary compounds for use within the invention may also be generated, for example, according to Routes 1,2,3, and 4 described herein, below. These reaction and synthetic schemes are provided for illustrative purposes only, and it is understood that abbreviated, alternate, and modified schemes, e.g, encompassing essential elements of these schemes, or their equivalents, are also contemplated within the scope of the invention.

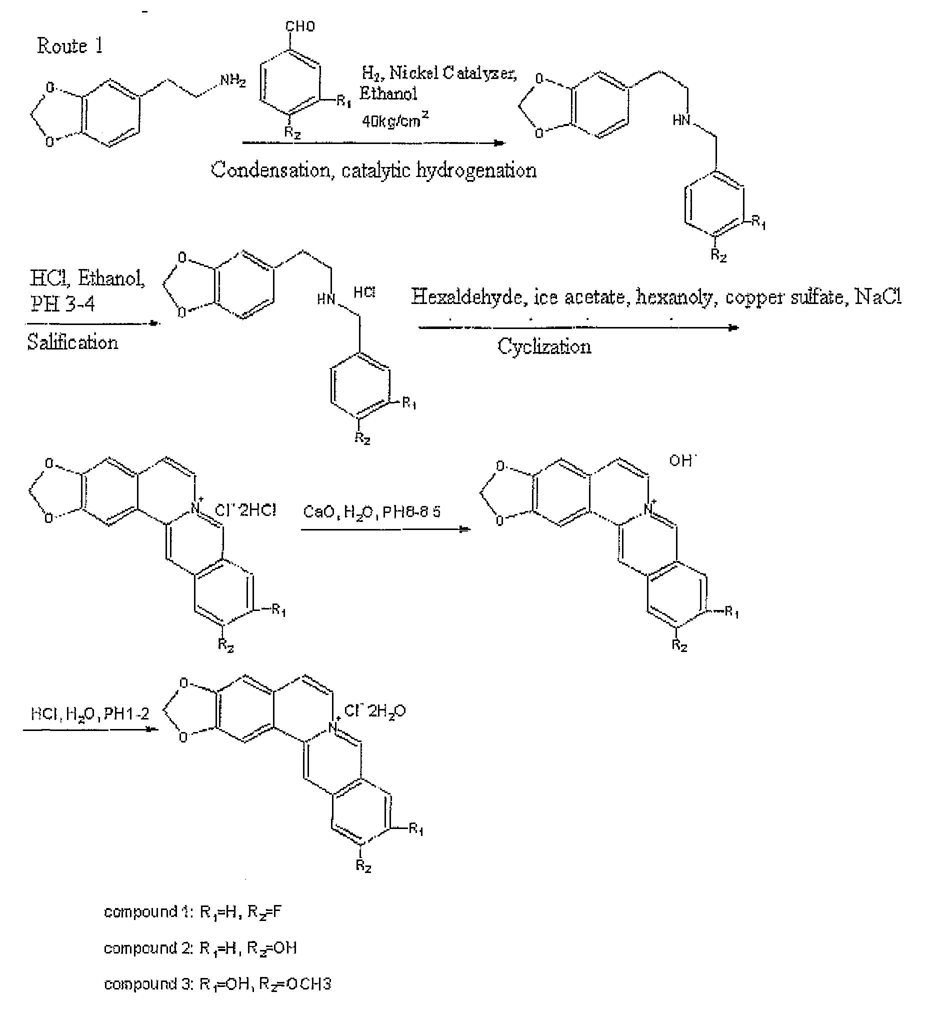

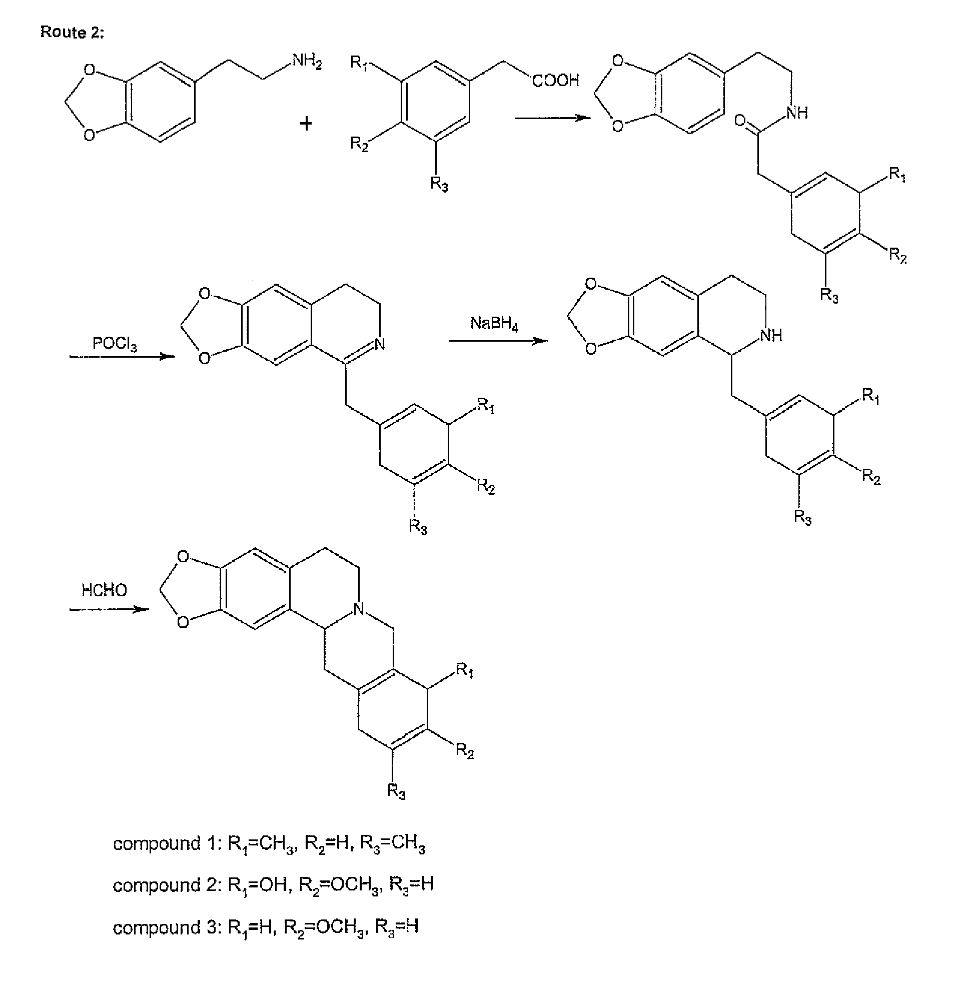

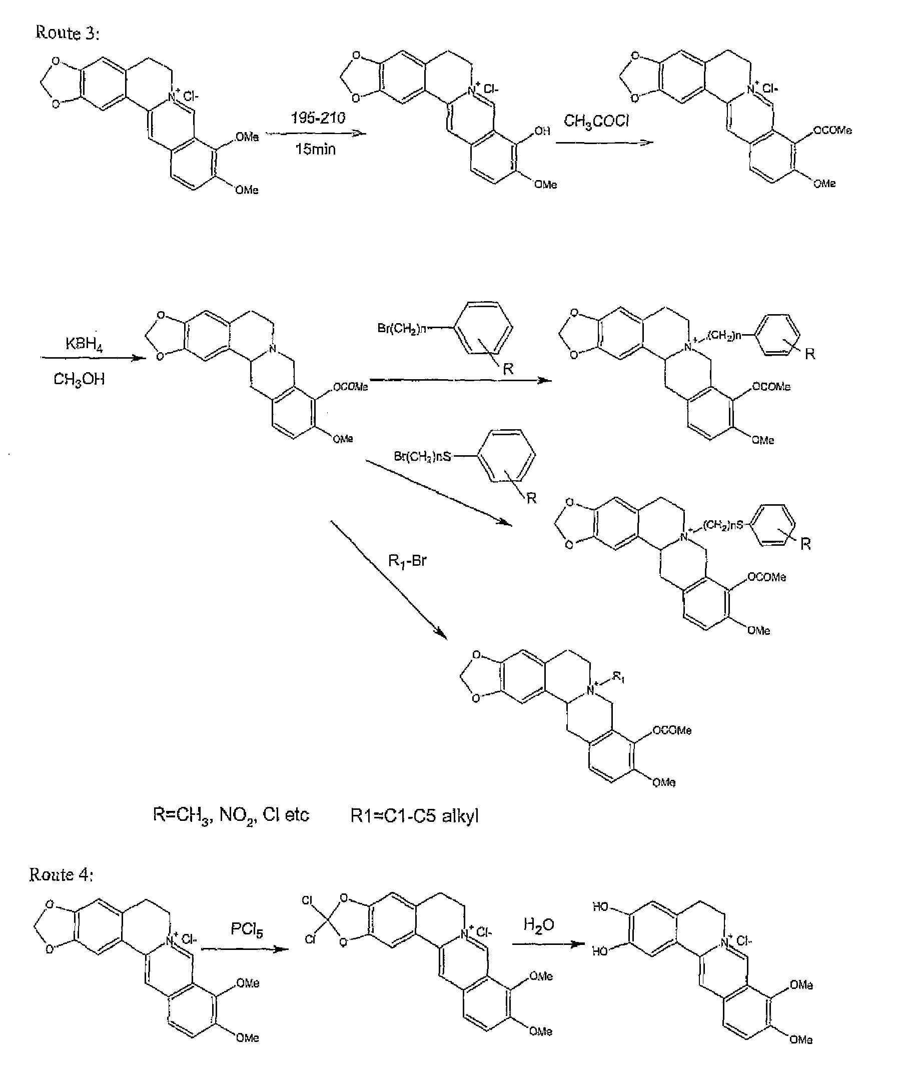

Lipid lowering compositions comprising a compound of formula I, including pharmaceutical formulations of the invention, comprise a lipid lowering effective amount of a berberine compound or berberine related or derivative compound of Formula I, which is effective for prophylaxis and/or treatment of hyperlipidemia and elevated cholesterol in a mammalian subject. Typically, a lipid lowering effective amount, including a cholesterol lowering effective amount, of a berberine compound or berberine related or derivative compound of Formula I will comprise an amount of the active compound which is therapeutically effective, in a single or multiple unit dosage form, over a specified period of therapeutic intervention, to measurably alleviate one or more symptoms of hyperlipidemia or elevated cholesterol in the subject, and/or to alleviate one or more symptom(s) of a cardiovascular disease or condition in the subject. Within exemplary embodiments, these compositions are effective within *in vivo* treatment methods to alleviate hyperlipidemia

Lipid lowering compositions of the invention typically comprise a lipid lowering effective amount or unit dosage of a berberine compound or berberine related or derivative compound of Formula I, which may be formulated with one or more pharmaceutically acceptable carriers, excipients, vehicles, emulsifiers, stabilizers, preservatives, buffers, and/or other additives that may enhance stability, delivery, absorption, half-life, efficacy, pharmacokinetics, and/or pharmacodynamics, reduce adverse side effects, or provide other advantages for pharmaceutical use. Lipid lowering effective amounts including cholesterol lowering effective amounts of a berberine compound or berberine related or derivative compound (eg, a unit dose comprising an effective concentration/amount of berberine, or of a selected pharmaceutically acceptable salt, isomer, enantiomer, solvate, polymorph and/or prodrug of berberine) will be readily determined by those of ordinary skill in the art, depending on clinical and patient-specific factors. Suitable effective unit dosage amounts of the active compounds for administration to mammalian subjects, including humans, may range from 10 to 1500 mg, 20 to 1000 mg, 25 to 750 mg, 50 to 500 mg, or 150 to 500 mg In certain embodiments, the anti hyperlipidemia or hypolipidemia effective dosage of a berberine compound or berberine related or derivative compound of Formula I maybe selected within narrower ranges of, for example, 10 to 25 mg, 30-50 mg, 75 to 100 mg, 100 to 250 mg, or 250 to 500 mg, These and other effective unit dosage amounts may be administered in a single dose, or in the form of multiple daily, weekly or monthly doses, for example in a dosing regimen comprising from 1 to 5, or 2-3, doses administered per day, per week, or per month In one exemplary embodiment, dosages of 10 to 25 mg, 30-50 mg, 75 to 100 mg, 100 to 250 mg, or 250 to 500 mg, are administered one, two, three, four, or five times per day. In more detailed embodiments, dosages of 50-75 mg, 100-200 mg, 250-400 mg, or 400-600 mg are administered once or twice daily In alternate embodiments, dosages are calculated based on body weight, and may be administered, for example, in amounts from about 0.5mg/kg to about 100mglkg per day, 1mg/kg to about 75mg/kg per day, 1mg/kg to about 50mg/kg per day, 2mg/kg to about 50mg/kg per day, 2mg/kg to about 30mg/kg per day or 3mg/kg to about 30mg/kg per day.

The amount, timing and mode of delivery of compositions of the invention comprising an anti -hyperlipidemia effective amount of a berberine compound or berberine related or derivative compound of Formula I will be routinely adjusted on an individual basis, depending on such factors as weight, age, gender, and condition of the individual, the acuteness of the hyperlipidemia and/or related symptoms, whether the administration is prophylactic or therapeutic, and on the basis of other factors known to effect drug delivery, absorption, pharmacokinetics, including half-life, and efficacy.

An effective dose or multi-dose treatment regimen for the instant lipid lowering formulations will ordinarily be selected to approximate a minimal dosing regimen that is necessary and sufficient to substantially prevent or alleviate hyperlipidemia and cardiovascular diseases in the subject, and/or to substantially prevent or alleviate one or more symptoms associated with hyperlipidemia in the subject. A dosage and administration protocol will often include repeated dosing therapy over a course of several days or even one or more weeks or years. An effective treatment regime may also involve prophylactic dosage administered on a day or multi-dose per day basis lasting over the course of days, weeks, months or even years.

Various assays and model systems can be readily employed to determine the therapeutic effectiveness of antihyperlipidemia treatment according to the invention For example, blood tests to measure total cholesterol as well as triglycerides, LDL and HDL levels are routinely given. Individuals with a total cholesterol level of greater than 200 mg/dL are considered borderline high risk for cardiovascular disease Those with a total cholesterol level greater than 239 mg/dL are considered to be at high risk. An LDL level of less than 100 mg/dL is considered optimal. LDL levels between 130 to 159mg/dL are borderline high risk. LDL levels between 160 to 189 mg/dL are at high risk for cardiovascular disease and those individuals with an LDL greater than 190 mg/dL are considered to be at very high risk for cardiovascular disease. Triglyceride levels of less than 150 mg/dL is considered normal. Levels between 150-199 mg/dL are borderline high and levels above 200 are considered to put the individual at high risk for cardiovascular disease. Lipid levels can be determined by standard blood lipid profile tests Effective amounts of the compositions of the invention will lower elevated lipid levels by at least 10%, 20%, 30%, 50% or greater reduction, up to a 75-90%, or 95% or greater. Effective amounts will also move the lipid profile of an individual towards the optimal category for each lipid, i.e, decrease LDL levels from 190mg/dl to within 130 to 159mg/dL or even further to below 100 mg/dL Effective amounts may further decrease LDL or triglyceride levels by about 10 to about 70 mg/dL, by about 20 to about 50 mg/dL, by about 20 to about 30 mg/dL, or by about 10 to about 20 mg/dL.

Individuals may also be evaluated using a hs-CRP (high-sensitivity G-zeactive protein) blood test. Those with a hs-CRP result of less than 10 mg/L are at low risk for cardiovascular disease. Individuals with a hs-CRP result between about 1.0 to 3 0 mg/L are at average risk for cardiovascular disease. Those with a hs-CRP result greater than 3.0 mg/L are at high risk of cardiovascular disease. Effective amounts of the compositions of the present invention will lower hs-CRP results below 30mg/L. Effective amounts of'the compositions of the present invention can lower hs-CRP results by about 0.5 to about 3.0mg/L, and further by about 0.5 to about 2.0mg/L

Effectiveness of the compositions and methods of the invention may also be demonstrated by a decrease in the symptoms of cardiovascular disease including shortness of breath, chest pain, leg pain, tiredness, confusion vision changes, blood in urine, nosebleeds, irregular heartbeat, loss of balance or coordination, weakness, or vertigo.

For each of the indicated conditions described herein, test subjects will exhibit a 10%, 20%, 30%, 50% or greater reduction, up to a 75-90%, or 95% or greater, reduction, in one or more symptom(s) caused by, or associated with, hyperlipidemia, elevated cholesterol and/or a targeted cardiovascular disease or condition in the subject, compared to placebo-treated or other suitable control subjects.

Within additional aspects of the invention, combinatorial lipid lowering formulations and coordinate administration methods are provided which employ an effective amount of a berberine compound or berberine related or derivative compound of Formula I and one or more secondary or adjunctive agent(s) that is/are combinatorially formulated or coordinately administered with the berberine compound or berberine related or derivative compound to yield a combined, multi-active agent anti-hyperlipidemia composition or coordinate treatment method. Exemplary combinatorial formulations and coordinate treatment methods in this context employ the berberine compound or berberine related or derivative compound in combination with the one or more secondary anti-hyperlipidemia agent(s), or with one or more adjunctive therapeutic agent(s) that is/are useful for treatment or prophylaxis of the targeted (or associated) disease, condition and/or symptom(s) in the selected combinatorial formulation or coordinate treatment regimen, for most combinatorial formulations and coordinate treatment methods of the invention, a berberine compound or berberine related or derivative compound of Formula I is formulated, or coordinately administered, in combination with one or more secondary or adjunctive therapeutic agent(s), to yield a combined formulation or coordinate treatment method that is combinatorially effective or coordinately useful to treat hyperlipidemia and/or one or more symptom(s) of'a cardiovascular disease or condition in the subject. Exemplary combinatorial formulations and coordinate treatment methods in this context employ a berberine compound or berberine related or derivative compound of Formula I in combination with one or more secondary or adjunctive therapeutic agents selected from, e.g., antihyperlipidemic agents; antidyslipidemic agents; plasma HDL- raising agents; antihypercholesterolemic agents, including, but not limited to, cholesterol-uptake inhibitors; cholesterol biosynthesis inhibitors, e.g., FIMG-CoA reductase inhibitors (also referred to as statins, such as lovastatin, simvastatin, pravastatin, fluvastatin, rosuvastatin, pitavastatin, and atorvastatin); HMG-CoA synthase inhibitors; squalene epoxidase inhibitors or squalene synthetase inhibitors (also known as squalene synthase inhibitors); acyl-coenzyme A cholesterol acyltransferase (ACAT) inhibitors, including, but not limited to, melinamide; probucol; nicotinic acid and the salts thereof; niacinamide; cholesterol absorption inhibitor, including, but not limited to, β-sitosterol or ezetimibe; bile acid sequestrant anion exchange resins, including, but not limited to cholestyramine, colestipol, colesevelam or dialkylaminoalkyl derivatives of a cross-linked dextran; LDL receptor inducers; fibrates, including, but not limited to, clofibrate, bezafibrate, fenofibrate and gemfibrozil; vitamin B6 (also known as pyridoxine) and the pharmaceutically acceptable salts thereof such as the HC1 salt; vitamin B12 (also known as cyanocobalamin); vitamin B3 (also known as nicotinic acid and niacinamide, supra); anti-oxidant vitamins, including, but not limited to, vitamin C and E and betacarotene; β blockers; angiotensin II receptor (AT₁) antagonist; angiotensin-converting enzyme inhibitors, rein inhibitors; platelet aggregation inhibitors, including, but not limited to, fibrinogen receptor antagonists, i.e, glycoprotein IIb/IIIa fibrinogen receptor antagonists; hormones, including but not limited to, estrogen; insulin; ion exchange resins; omega-3 oils; benfluorex; ethyl icosapentate; and amlodipine. Adjunctive therapies may also include increases or changes in exercise, surgery, and changes in diet (e.g., toward a lower cholesterol and/or increased fiber diet). As noted above, herbal preparations (e.g., solid preparations, liquid extracts, etc.) will also be effectively employed in combinatorial formulations and coordinate treatment protocols to reduce lipid levels. Effective combinatorial herbal agents in this context include, for example, curcumin, anti-oxidant vitamins, gugulipid (from resin of Commiphora Mukul tree), garlic, vitamin E, soy, soluble fiber, fish oil, green tea, carnitine, chromium coenzyme Q10, grape seed extract, pantothine, red yeast rice, and royal jelly.

In certain embodiments the invention provides combinatorial lipid lowering formulations comprising berberine and one or more adjunctive agent(s) having antiinflammatory or lipid lowering activity Within such combinatorial formulations, berberine and the adjunctive agent(s) having lipid lowering activity will be present in a combined formulation in lipid lowering effective amounts, alone or in combination. In exemplary embodiments, berberine and a non-berberine lipid lowering agent(s) will each be present in a lipid lowering amount (i.e., in singular dosage which will alone elicit a detectable anti-hyperlipidemia response in the subject). Alternatively, the combinatorial formulation may comprise one or both of the berberine and non-berberine agents in sub-therapeutic singular dosage amount(s), wherein the combinatorial formulation comprising both agents features a combined dosage of both agents that is collectively effective in eliciting an lipid lowering response. Thus, one or both of the berberine and non-berberine agents maybe present in the formulation, or administered in a coordinate administration protocol, at a sub-therapeutic dose, but collectively in the formulation or method they elicit a detectable lipid lowering response in the subject.

To practice coordinate administration methods of the invention, a berberine compound or berberine related or derivative compound of Formula I may be administered, simultaneously or sequentially, in a coordinate treatment protocol with one or more of the secondary or adjunctive therapeutic agents contemplated herein. Thus, in certain embodiments a berberine compound or berberine related or derivative compound is administered coordinately with a non-berberine, lipid lowering agent, or any other secondary or adjunctive therapeutic agent contemplated herein, using separate formulation or a combinatorial formulation as described above (i.e, comprising both a berberine compound or berberine related or derivative compound, and a non-berberine therapeutic agent), this coordinate administration may be done simultaneously or sequentially in either order, and there may be a time period while only one or both (or all) active therapeutic agents individually and/or collectively exert their biological activities. A distinguishing aspect of all such coordinate treatment methods is that the berberine compound or berberine related or derivative compound exerts at least some lipid lowering activity, which yields a favorable clinical response in conjunction with a complementary lipid lowering, or distinct, clinical response provided by the secondary or adjunctive therapeutic agent. Often, the coordinate administration of the berberine compound or berberine related or derivative compound with the secondary or adjunctive therapeutic agent will yield improved therapeutic or prophylactic results in the subject beyond a therapeutic effect elicited by the berberine compound or berberine related or derivative compound, or the secondary or adjunctive therapeutic agent administered alone. This qualification contemplates both direct effects, as well as indirect effects.

Within exemplary embodiments, a berberine compound or berberine related or derivative compound of Formula I will be coordinately administered (simultaneously or sequentially, in combined or separate formulation(s)), with one or more secondary lipid lowering agents, or other indicated therapeutic agents, e.g., selected from, for example, cholesterol-uptake inhibitors; cholesterol biosynthesis inhibitors, e.g., HMG-CoA reductase inhibitors (also referred to as statins, such as lovastatin, simvastatin, pravastatin, fluvastatin, rosuvastatin, pitavastatin, and atorvastatin); HMG-CoA synthase inhibitors; squalene epoxidase inhibitors or squalene synthetase inhibitors (also known as squalene synthase inhibitors); acyl-coenzyme A cholesterol acyltransferase (ACAT) inhibitors, including, but not limited to, melinamide; probucol; nicotinic acid and the salts thereof; niacinamide; cholesterol absorption inhibitors, including, but not limited to, β-sitosterol or ezetimibe; bile acid sequestrant anion exchange resins, including, but not limited to cholestyramine, colestipol, colesevelam or dialkylaminoalkyl derivatives of a cross-linked dextran; LDL receptor inducers; fibrates, including, but not limited to, clofibrate, bezafibrate, fenofibrate and gemfibrozil; vitamin B6 (also known as pyridoxine) and the pharmaceutically acceptable salts thereof; such as the HCl salt; vitamin B12 (also known as cyanocobalamin); vitamin B3 (also known as nicotinic acid and niacinamide, supra); anti-oxidant vitamins, including, but not limited to, vitamin C and E and betacarotene; β blockers; angiotensin II receptor (AT₁) antagonist; angiotensin-converting enzyme inhibitors, renin inhibitors; platelet aggregation inhibitors, including, but not limited to, fibrinogen receptor antagonists, i.e., glycoprotein IIb/IIIa fibrinogen receptor antagonists; hormones, including but not limited to, estrogen; insulin; ion exchange resins; omega-3 oils; benfluorex; ethyl icosapentate; and amlodipine. Adjunctive therapies may also include increases in exercise, surgery, and changes in diet (e.g., to a low cholesterol diet). Some herbal remedies may also be empoyed effectively in combinatorial formulations and coordinate therapies for treating hyperlipidemia, for example curcumin, gugulipid, garlic, vitamin E, soy, soluble fiber, fish oil, green tea, carnitine, chromium, coenzyme Q10, anti-oxidant vitamins, grape seed extract, pantothine, red yeast rice, and royal jelly.

As noted above, in all of the various embodiments of the invention contemplated herein, the anti hyperlipidemia and related methods and formulations may employ a berberine, compound or berberine related or derivative compound of Formula I in any of a variety of forms, including any one or combination of the subject compound's pharmaceutically acceptable salts, isomers, enantiomers, polymorphs, solvates, hydrates, and/or prodrugs. In exemplary embodiments of the invention, berberine is employed within the therapeutic formulation and methods for illustrative purposes.

The pharmaceutical compositions of the present invention may be administered by any means that achieve their intended therapeutic or prophylactic purpose. Suitable routes of administration for the compositions of'the invention include, but are not limited to, oral, buccal, nasal, aerosol, topical, transdermal, mucosal, injectable, slow release, controlled release, iontophoresis, sonophoresis, and including all other conventional delivery routes, devices and methods. Injectable methods include, but are not limited to, intravenous, intramuscular, intraperitoneal, intraspinal, intrathecal, intracerebroventricular, intraarterial, subcutaneous and intranasal routes.

The compositions of the present invention may further include a pharmaceutically acceptable carrier appropriate for the particular mode of administration being empoyed. Dosage forms of the compositions of the present invention include excipients recognized in the art of pharmaceutical compounding as being suitable for the preparation of' dosage units as discussed above, Such excipients include, without intended limitation, binders, fillers, lubricants, emulsifiers, suspending agents, sweeteners, flavorings, preservatives, buffers, wetting agents, disintegrants, effervescent agents and other conventional excipients and additives.

If desired, the compositions of the invention can be administered in a controlled release form by use of a slow release carrier, such as a hydrophilic, slow release polymers. Exemplary controlled release agents in this context include, but are not limited to, hydroxypropyl methyl cellulose, having a viscosity in the range of about 100 cps to about 100,000 cps or other biocompatible matrices such as cholesterol.

Compositions of the invention will often be formulated and administered in an oral dosage form, optionally in combination with a carrier or other additive(s). Suitable carriers common to pharmaceutical formulation technology include, but are not limited to, microcrystalline cellulose, lactose, sucrose, fructose, glucose, dextrose, or other sugars, di-basic calcium phosphate, calcium sulfate, cellulose, methylcellulose, cellulose derivatives, kaolin, mannitol, lactitol, maltitol, xylitol, sorbitol, or other sugar alcohols, dry starch, dextrin, maltodextrin or other polysaccharides, inositol, or mixtures thereof. Exemplary unit oral dosage forms for use in this invention include tablets, which may be prepared by any conventional method of preparing pharmaceutical oral unit dosage forms can be utilized in preparing oral unit dosage forms, Oral unit dosage forms, such as tablets, may contain one or more conventional additional formulation ingredients, including, but not limited to, release modifying agents, glidants, compression aides, disintegrants, lubricants, binders, flavors, flavor enhancers, sweeteners and/or preservatives. Suitable lubricants include stearic acid, magnesium stearate, talc, calcium stearate, hydrogenated vegetable oils, sodium benzoate, leucine carbowax, magnesium lauryl sulfate, colloidal silicon dioxide and glyceryl monostearate. Suitable glidants include colloidal silica, fumed silicon dioxide, silica, talc, fumed silica, gypsum and glyceryl monostearate Substances which may be used for coating include hydroxypropyl cellulose, titanium oxide, talc, sweeteners and colorants.

Additional compositions of the invention can be prepared and administered in any of a variety of inhalation or nasal delivery forms known in the art. Devices capable of depositing aerosolized purified berberine formulations in the sinus cavity or pulmonary alveoli of a patient include metered dose inhalers, nebulizers, dry powder generators, sprayers, and the like. Methods and compositions suitable for pulmonary delivery of drugs for systemic effect are well known in the art. Additional possible methods of delivery include deep lung delivery by inhalation. Suitable formulations, wherein the carrier is a liquid, for administration, as for example, a nasal spray on as nasal drops, may include aqueous or oily solutions of berberine composition and any additional active or inactive ingredient(s).

Further compositions and methods of the invention are provided for topical administration of a berberine compound or berberine related or derivative compound for the treatment of hyperlipidemia. Topical compositions may comprise a berberine compound or berberine related or derivative compound of Formula I along with one or more additional active or inactive component(s) incorporated in a dermatological or mucosal acceptable earner, including in the form of aerosol sprays, powders, dermal patches, sticks, granules, creams, pastes, gels, lotions, syrups, ointments, impregnated sponges, cotton applicators, or as a solution or suspension in an aqueous liquid, non-aqueous liquid, oil-in-water emulsion, or watei-in-oil liquid emulsion. These topical compositions may comprise a berberine compound or berberine related or derivative compound of Formula I dissolved or dispersed in a portion of a water or other solvent or liquid to be incorporated in the topical composition or delivery device. It can be readily appreciated that the transdermal route of administration may be enhanced by the use of a dermal penetration enhancer known to those skilled in the art. Formulations suitable for such dosage forms incorporate excipients commonly utilized therein, particularly means, e.g., structure or matrix, for sustaining the absorption of the drug over an extended period of time, for example, 24 hours Transdermal delivery may also be enhanced through techniques such as sonophoresis.

Yet additional berberine compositions of the invention are designed for parenteral administration, e g. to be administered intravenously, intramuscularly, subcutaneously or intraperitoneally, including aqueous and non-aqueous sterile injectable solutions which, like many other contemplated compositions of the invention, may optionally contain anti-oxidants, buffers, bacteriostats and/or solutes which render the formulation isotonic with the blood of the mammalian subject; and aqueous and non-aqueous sterile suspensions which may include suspending agents and/or thickening agents. The formulations may be presented in unit-dose or multi-dose containers. Additional compositions and formulations of the invention may include polymers for extended release following parenteral administration. The parenteral preparations may be solutions, dispersions or emulsions suitable for such administration. The subject agents may also be formulated into polymers for extended release following parenteral administration. Pharmaceutically acceptable formulations and ingredients will typically be sterile or readily sterilizable, biologically inert, and easily administered. Such polymeric materials are well known to those of ordinary skill in the pharmaceutical compounding arts. Parenteral preparations typically contain buffering agents and preservatives, and injectable fluids that are pharmaceutically and physiologically acceptable such as water, physiological saline, balanced salt solutions, aqueous dextrose, glycerol or the like. Extemporaneous injection solutions, emulsions and suspensions may be prepared from sterile powders, granules and tablets of the kind previously described. Preferred unit dosage formulations are those containing a daily dose or unit, daily sub-dose, as described herein above, or an appropriate fraction thereof; of the active ingredient(s).

In more detailed embodiments, compositions of the invention may comprise a berberine compound or berberine related or derivative compound of Formula I encapsulated for delivery in microcapsules, microparticles, or microspheres, prepared, for example, by coacervation techniques or by interfacial polymerization, for example, hydroxymethylcellulose or gelatin-microcapsules and poly(methylmethacylate) microcapsules, respectively; in colloidal drug delivery systems (for example, liposomes, albumin microspheres, microemulsions, nanoparticles and nanocapsules); or within macroemulsions.

As noted above, in certain embodiments the methods and compositions of the invention may employ pharmaceutically acceptable salts, e.g., acid addition or base salts of the above-described berberine compounds and/or berberine related or derivative compounds. Examples of pharmaceutically acceptable addition salts include inorganic and organic acid addition salts. Suitable acid addition salts are formed from acids which form non-toxic salts, for example, hydrochloride, hydrobromide, hydroiodide, sulphate, hydrogen sulphate, nitrate, phosphate, and hydrogen phosphate salts. Additional pharmaceutically acceptable salts include, but are not limited to, metal salts such as sodium salts, potassium salts, cesium salts and the like; alkaline earth metals such as calcium salts, magnesium salts and the like; organic amine salts such as triethylamine salts, pyridine salts, picoline salts, ethanolamine salts, triethanolamine salts, dicyclohexylamine salts, N,N'-dibenzylethylenediamine salts and the like; organic acid salts such as acetate, citrate, lactate, succinate, tartrate, maleate, fumarate, mandelate, acetate, dichloroacetate, trifluoroacetate, oxalate, and formate salts; sulfonates such as methanesulfonate, benzenesulfonate, and p-toluenesulfonate salts; and amino acid salts such as arginate, asparginate, glutamate, tartrate, and gluconate salds. Suitable base salts are formed from bases that form non-toxic salts, for example aluminum, calcium, lithium, magnesium, potassium, sodium, zinc and diethanolamine salts.

To illustrate the range of useful salt forms of berberine compounds and berberine related and derivative compounds within the methods and compositions of the invention, an exemplary assemblage of salt forms of berberine were produced and tested for their solubility (Table 2) The novel berberine salts thus provided embody yet additional aspects of the invention and exemplify the broad assemblage of useful berberine and related compounds herein.

**Table 2. Exemplary Berberine Salts**

| Sample Code | Solvent | Amount of Solute (mg) | Amount of Solvent | Solubility Ranking |
|---|---|---|---|---|
| 1 Citrate | Distill Water | 10 | 75 | Slightly Soluble |
| 2 Cysteine | Distill Water | 10 | 88 | Slightly Soluble |
| 3 Acetate | Distill Water | 10 | 9.0 | Slightly Soluble |
| 4 Lactate | Distill Water | 10 | 60 | Slightly Soluble |
| 5 Nitrate | Distill Water | 10 | 80 | Slightly Soluble |
| 6 Methanesulfonate | Distill Water | 10 | 1 5 | Slightly Soluble |
| 7 Hydrosulfate* | Distill Water | 10 | 15 | Slightly Soluble |
| 8 Sulfate* | Distill Water | 10 | 0 5 | Slightly Soluble |
| 9 Salicylate | Distill Water | 10 | 6 5 | Slightly Soluble |
| 10 Oxalate | Distill Water | 10 | 6 0 | Slightly Soluble |
| 11 Phosphate | Distill Water | 10 | 8 0 | Slightly Soluble |
| 12 Formate | Distill Water | 10 | 8.5 | Slightly Soluble |
| 13 Benzoate | Distill Water | 10 | 70 | Slightly Soluble |
| 14 Tartrate | Distill Water | 10 | 70 | Slightly Soluble |
| 15 Toluenesulfonate | Distill Water | 10 | 11.0 | Extremely Sightly Soluble |
| 16 Trifluoroacelate | Distill Water | 10 | 7 5 | Slightly Soluble |
| 17 Control: Hydrochloric | Distill Water | 10 | 10.0 | Extremely Slightly Soluble |

In other detailed embodiments, the methods and compositions of the invention for employ prodrugs of berberine compounds or berberine related or derivative compounds of Formula I. Prodrugs are considered to be any covalently bonded carriers which release the active parent drug in vivo. Examples of prodrugs useful within the invention include esters or amides with hydroxyalkyl or aminoalkyl as a substituent, and these may be prepared by reacting such compounds as described above with anhydrides such as succinic anhydride.

The invention disclosed herein will also be understood to encompass methods and compositions comprising a berberine compound or berberine related or derivative compounds of Formula I using in vivo metabolic products of the said compounds (either generated *in vivo* after administration of the subject precursor compound, or directly administered in the form of the metabolic product itself). Such products may result for example from the oxidation, reduction, hydrolysis, amidation, esterification and the like of the administered compound, primarily due to enzymatic processes. Accordingly, the invention includes methods and compositions of the invention employing compounds produced by a process comprising contacting a berberine compound or berberine related or derivative compound of Formula I with a mammalin subject for a period of time sufficient to yield a metabolic product thereof. Such products typically are identified by preparing a radiolabelled compound of the invention, administering it parenterally in a detectable dose to an animal such as rat, mouse, guinea pig, monkey, or to man, allowing sufficient time for metabolism to occur and isolating its conversion products from the urine, blood or other biological samples.

The invention disclosed herein will also be understood to encompass diagnostic compositions for diagnosing the risk level, presence, severity, or treatment indicia of, or otherwise managing a hyperlipidemia and/or cardiovascular disease or condition in a mammalian subject, comprising contacting a labeled (e.g., isotopically labeled, fluorescent labeled or otherwise labeled to permit detection of the labeled compound using conventional methods) berberine compound or berberine related or derivative compound of Formula I to a mammalian subject (e.g., to a cell, tissue, organ, or individual) at risk or presenting with one or more symptom(s) of hyperlipidemia and/or cardiovascular disease, and thereafter detecting the presence, location, metabolism, and/or binding state (e.g., detecting binding to an unlabeled binding partner involved in LDL receptors physiology/metabolism) of the labeled compound using any of a broad array of known assays and labeling/detection methods. In exemplary embodiments, a berberine compound or berberine related or derivative compound of Formula I is isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine and chloride, such as ²H, ³H, ¹³C ¹⁴C, ¹⁵N, ¹⁸O, ¹⁷O, ³¹P, ³²P, ³⁵S, ¹⁸F, and ³⁶Cl respectively The isotopically-labeled copmpound is then administered to an individual or other subject and subsequently detected as described above, yielding useful diagnostic and/or therapeutic management data, according to conventional techniques.

### Examples

The experiments described below demonstrate novel and powerful uses for a berberine compounds and berberine related and derivative compounds as cholesterol lowering drugs that can effectively lowers serum cholesterol, triglycerides and LDL through a mechanism other than that used by current hypolipidemic drugs, such as statins. In exemplary experiments, cells from a human hepatoma-derived cell line, HepG2, were treated for 24 hours with 700 compounds isolated from Chinese herbs. RNA was then isolated from the cells and analysis of LDLR mRNA was determined using semi-quantitative RT-PCR assays. Of the compounds tested, berberine demonstrated the greatest increase in LDLR expression. Treating HepG2 cells cultured in medium containing 0 5% lipoprotein-depleted fetal bovine serum or serum supplemented with sterols and berberine caused time dependent increases in the expression of LDLR mRNA. These and additional findings are further expanded and elucidated within the following examples

### Example I

### Effects of Berberine on the levels of cholesterol, triglycerides and LDL protein in a hyperlipidemia Chinese Hamster

Two weeks prior to treatment, female Chinese hamsters purchased from the National Institute of Vaccine and Serum research (Beijing, China) were switched to a high fat and cholesterol diet (10% lard, 10% egg yolk powder and 1% cholesterol) After two weeks, groups of 14 hamsters were given either 10 mg/kg/day of berberine through peritoneal injection, 20 mg/kg/day of berberine through peritoneal injection, 50 mg/kg/day, 100 mg/kg/day of berberine orally or saline for ten days. Serum cholesterol, triglyceride and LDL levels were measured after 4 h fasting before, during and after the course of the treatment. Four hours after the course of the treatment, the animals were sacrificed and their livers removed for analysis.

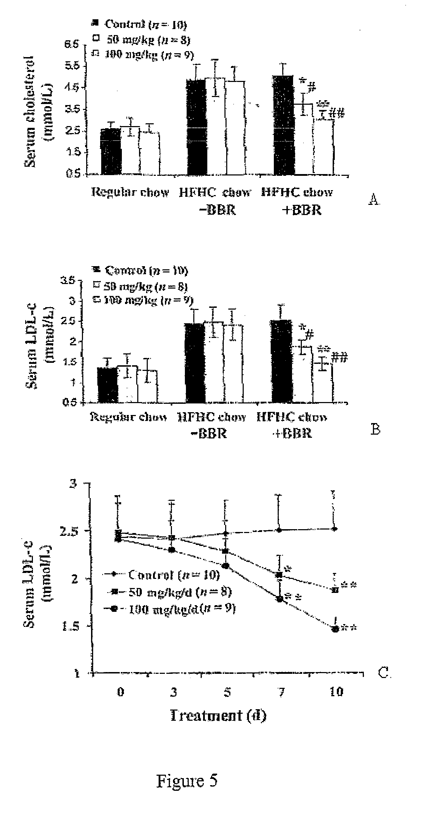

As can be seen in Table 3, berberine decreased the levels of cholesterol triglycerides and LDL protein in all of the treated animals After the 10 day treatment, a dose of 50/mg/kg/day of berberine reduced LDL by 26% and a dose of 100 mg/kg/day reduced LDL by 42% Reductions in serum LDL were observed by day 5 and became significant by day 7 at both doses (Figure 5).

**Table 3: Lipid lowering effects of berberine in hyperlipidemia Chinese hamster**

| Treatment | | n | Cholesterol | Triglycerides | LDL Protein |
|---|---|---|---|---|---|
| Saline | (control group) | 14 | 64±10 | 3.6±04 | 28±09 |
| Berberine | 10mg/kg/day (peritoneal injection) | 14 | 41±07** | 2.6±0.3 | 16±0 3** |
| | 20mg/kg/day (peritoneal injection) | 14 | 3 2±0.5*** | 1 7±0 5* | 0.9±01*** |
| | 50mg/kg/day | 8 | 3.5±0.5 | | 2.07±0.9 |
| | 100mg/kg/day (oial) | 14 | 3.8±07** | 1 9±0 4* | 1 2±0 2* |

| | | | | | |
|---|---|---|---|---|---|
| *P<0 05; **P<0 01; ***P<0 001 (compared to the control group) | | | | | |

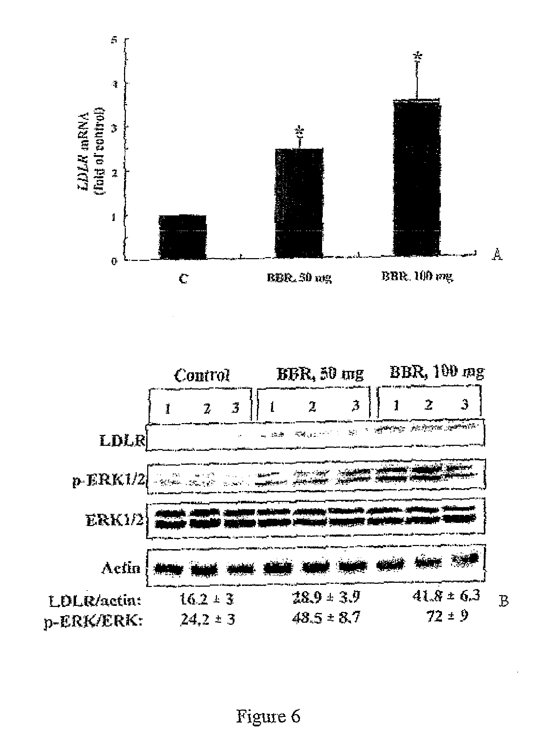

At the end of treatment, three animals from each group were killed and liver LDLR mRNA and protein expressions were examined by quantitative real-time RT-PCR and western blot analysis. For the real-time RI-PCR, reverse transcription with random primers using Superscript II at 42°C for 30 minutes with 1 µg of total RNA was performed using the ABI Prism 7900-HT Sequence Detection System and Universal MastexMix (Applied Biosystems, Poster City, CA) LDLR and GAPD mRNA expression levels were determined using the human LDLR and GAPD Pre-developed TaqMan Assay Reagents (Applied Biosystems). As can be seen in Figure 6, LDLR mRNA and protein levels were elevated in all berberine treated hamsters in a dose dependent manner. There was a 3.5 fold increase in mRNA and a 2.6 fold increase in protein in hamster livers treated with 100 mg/kg/day of berberine

### Example II

### Effects of berberine in humans with hyperlipidemia

Human patients with hyperlipidemia (52 males and 39 females) were randomly divided into two groups and treated with either 0.5g of berberine hydrochloride twice a day (n=63) or a placebo (n=28) for three months. After three months, fasting serum concentrations of cholesterol, triglycerides, HDL and LDL were measured using standard blood lipid tests. Liver and kidney functions were also measured. Those treated with berberine had statistically significant lower cholesterol, triglycerides and LDL protein levels than those treated with the placebo, with berberine hydrochloride lowering serum levels of cholesterol by 18% (P<0.001), triglycerides by 28% (P<0.001) and LDL by 20% (P<0.001). Because some participants were taking other medications that could have influenced the results, the results were reanalyzed using only the data from those participants who were neither on drugs nor special diets before or during berberine therapy. As can be seen in Table 4, the results of those who were only taking berberine hydrochloride were even more significant with serum levels of cholesterol decreasing by 29% (P< 0.0001), triglycerides by 35% (P<0.0001) and LDL by 25% (P<0.0001). Berberine was well tolerated by all subjects and no side effects were observed with the exception of one patient having mild constipation during treatment, which was relieved after reducing the dose to 0 25 g twice per day. BBR did not change kidney functions (as determined by measurements of creatine, blood urea nitrogen, and total bilirubin in treated and placebo subjects), but substantially improved liver function-reducing levels of alanine aminotransaminase, aspartate aminotransaminase, and gama glutamyl transpeptidase, by approximately 48%, 36%, and 41%, respectively. The placebo group showed no significant changes in these parameters.

**Table 4: Lipid lowering effects of berberine in hyperlipidemia patients**

| | Berberine treatment | Berberine Group^{a} | Placebo Group |
|---|---|---|---|
| | (3 months) | (n=32) | (n=11) |
| Serum level of cholesterol | | >5/2 mmol/L | >5 2 mmol/L |
| Cholesterol | Before | 5 9±0.7 | 6 0±08 |
| (mmol/L) | | | |
| | After | 4.2±0.9* | 5.8 ± 0.6 |
| Triglycerides | Before | 23±1.8 | 2.2±07 |
| (mmol/L) | | | |
| | After | 1.5±0.9* | 2.0±1.0 |
| LDL Protein | Before | 3 2±0 7 | 3.7±0 7 |
| (mmol/L) | | | |
| | After | 2.4±0.6*** | 3.7±0.8 |
| HDL Protein | Before | 1.1±0.3 | 1.2±0.5 |
| | After | 1.1±0.3 | 1.2±0.4 |

| | | | |
|---|---|---|---|
| ^{a}Statistical analysis of the baselines of cholesterol, trigylceride, HDL-c, and LDL-c showed that there were no significant differences between the berberine and placebo groups before therapy (p>0 05) ***P<0 0001 as compared to baselines of before treatment group (matched t test) | | | |

### Example III

### The effect of berberine on LDLR expression

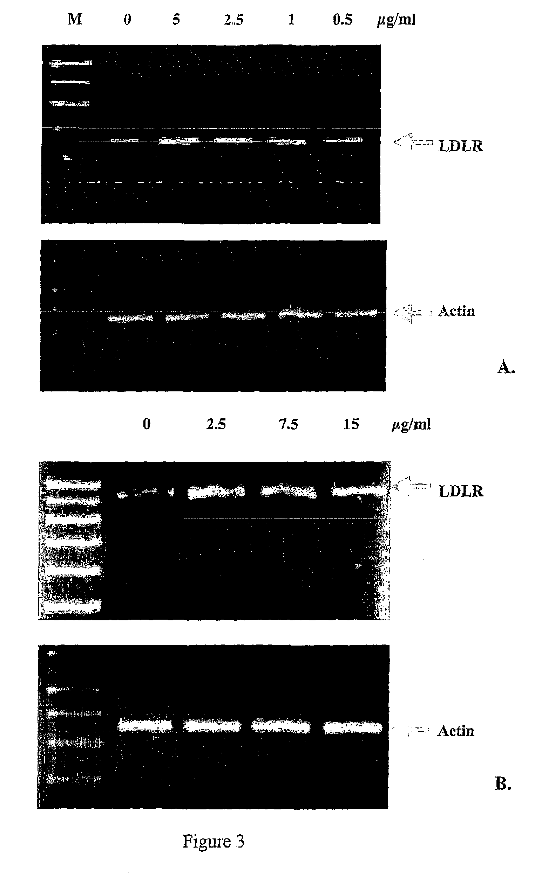

Bel-7402 cells were treated with 0, 0.5, 1, 2.5, 5, µg/ml of berberine or 2.5, 7.5 and 15 µg/ml of berberine sulfate. The cells were then centrifuged and washed and LDLR mRNA was extracted. LDLR mRNA levels were then measured using scan quantitative RT-PCR, (Figure 3 A and B). As can be seen in Figure 3A and B, treatment with berberine and berberine sulfate increased LDLR mRNA expression in a dose dependent fashion with 5µg/ml berberine increasing LDLR mRNA expression 2 3 fold Berberine also increased LDLR protein expression on the surface of BEL-7402 cells.

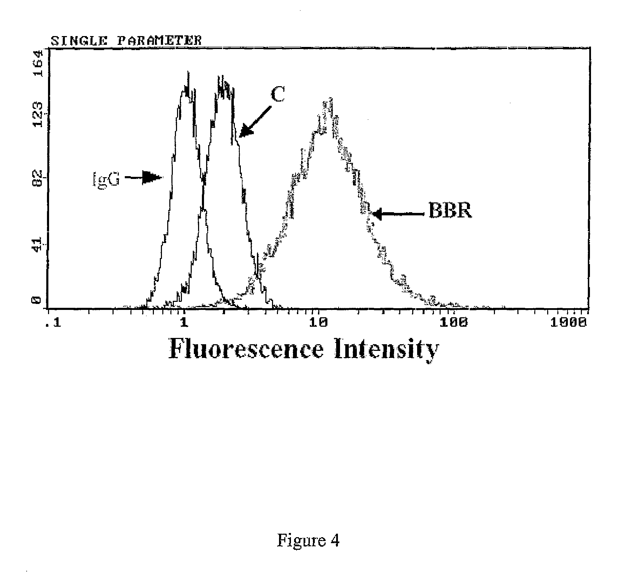

Bel-7402 cells treated with 5 µg/ml of berberine were detached with cell removal buffer containing EDIA, washed and resuspended in FACTS solution (PBS with 0.5% BSA and 0 02% sodium azide) at a density of 1 x 10⁶ cells/ml. Cells were then incubated with monoclonal antibody to LDLR (Santa Cruz Biotechnology, Inc., Santa Cruz, CA) at a final dilution of 1:50 and left at room temperature for 1 hour The cells were then reacted with isotope matched, nonspecific mouse IgG as a control for nonspecific staining. The cells were then washed and stained with FITC conjugated goat antibody to mouse IgG (Santa Cruz Biotechnology, Inc., Santa Cruz, CA, 1:100 dilution) and the fluorescence intensity was analyzed by FACS (FACSort, Becton Dickinson, Franklin Lakes, NJ) As can be seen in Figure 4, berberine increased cell surface LDLR protein expression 4 times.

The above studies demonstrate that berberine's serum lipid lowering effect is mediated through an increase in LDLR expression.

### Example IV

### Use of berberine and simvastatin in combination to lower serum lipid levels in rats

Rats were fed a high fat high cholesterol (HFHC) diet for 10 days, and then divided into groups of seven. The rats were then administered berberine or simvastatin, or a combination of berberine and simvastatin orally for 25 days. After 25 days, serum cholesterol, triglyceride and LDL-c levels were measured As can be seen in Table 5, treatment with berberine significantly decreased the cholesterol, triglycerides and LDL-c levels in the rats and was more effective than simvastatin in lowering triglyceride and LDL-c levels The combination of simvastatin, and berberine lowered the cholesterol, triglyceride and LDL-c levels further than either alone

**Table 5: Combination treatment with berberine and simvastatin in rats**

| Experiment Group | N | Daily dose (oral, mg/kg) | Total Cholesterol (mmol/L) | Triglyceride (mmol/L) | LDL-c (mmol/L) |
|---|---|---|---|---|---|
| Normal Control Group | 7 | no | 2.6±0 4 | 1.9±0 6 | 1.4±0 3 |
| Untreated hyper-lipidemia Group | 7 | no | 6 4±0 6 | 3.2±0.3 | 2.4±0.3 |
| Berberine Treatment Group | 7 | 100 | 3.8±0 5 | 21±0.3 | 1.2±0.2 |
| Simvastatin Treatment Group | 7 | 25 | 3.6±0 4 | 2.5±0 3 | 1.4±0 1 |
| Berberine+Simvastatin Group | 7 | 100+25 | 2.5±0 4 | 1.6±0 2 | 1.1±0 2 |

After 25 days, blood total cholesterol, triglyceride and LDL-c levels were examined. The result in tables are average ± standard error.

### Example V

### Use of berberine to increase LDLR mRNA stability

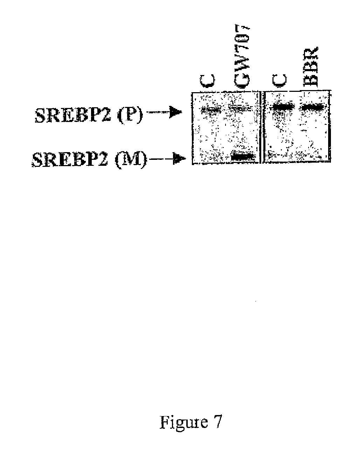

HepG2 cells were cultured with either berberine hydrochloride or GW707 as a positive control for 8 hours. Total cell lysates from untreated cells or cells treated with either berberine or GW707 were then harvested and analyzed by Western blot. As can be seen in Figure 7, GW70 substantially increased the amount of the mature form of SREB-2, whereas berberine had no effect. These data indicate that berberine effectively increases LDLR expression by a mechanism distinct from that used by statins, thereby further evincing that this novel drug and its related and derivative compounds will provide useful anti-hyperlipidemic formulations and methods with minimal side effects attributed to other known anti hyperlipidemic drugs.

### Example VI

### Function of berberine in the presence of statins

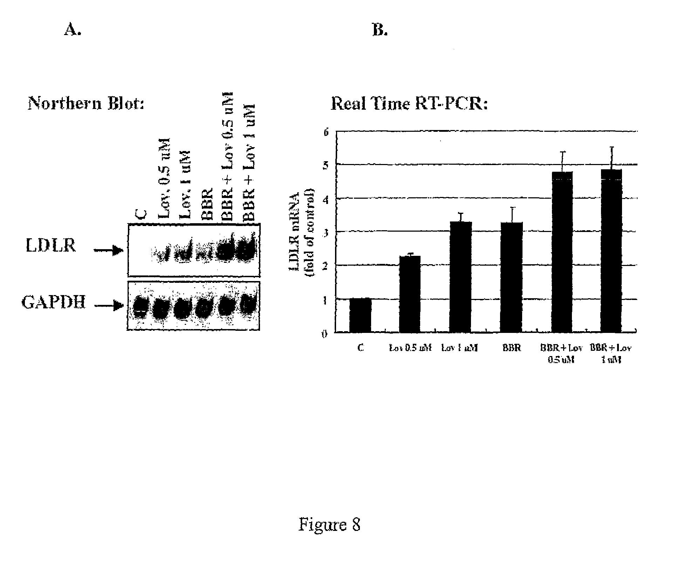

HepG2 cells were cultured in LPDS medium and were then untreated, treated with lovastatin at 0.5 and 1 µM concentrations with or without berberine for 24 hours, or were treated with berberine alone As can be seen in Figure 8, berberine and lovastatin had additive stimulation effects on LDLR mRNA expression, which data evince general utility of the novel, combinatorial formulations and coordinate treatment methods describe herein above.

### Example VII

### Analysis of LDLR promoter activity

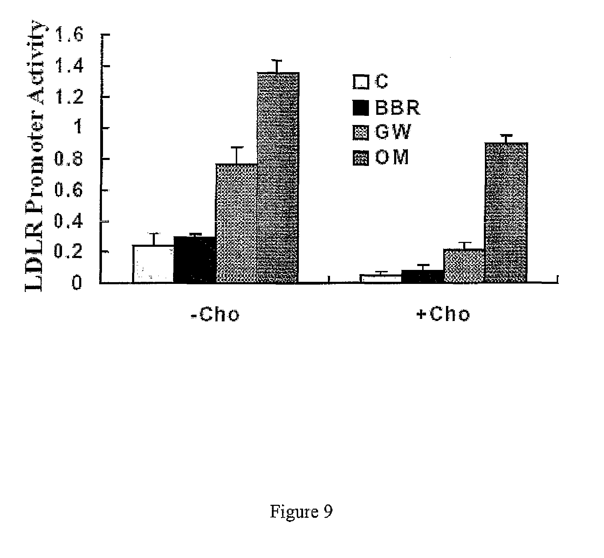

HepG2 cells were transfected with the reporter construct pLDLR234Luc, which contains the SRE-1 motif and the sterol-independent regulatory element that mediates the cytokine oncostatin M-induced transcription of the LDLR gene. After transfection, cells were culture in 0.5% lipoprotein depleted fetal bovine serum (LPDS) or LPDS and cholesterol medium followed by an 8 hour treatment with berberine, GW707 or oncostatin M. As can be seen in Figure 9, LDLR promoter activity was strongly elevated by GW707 and oncostatin M under both culturing conditions. Berberine had no effect, further evincing that this compound operates via a different mechanism of LDLR regulation compared to other known drugs possessing anti-hyperlipidemic activity.

### Example VIII

### Stabilization of LDLR mRNA by berberine

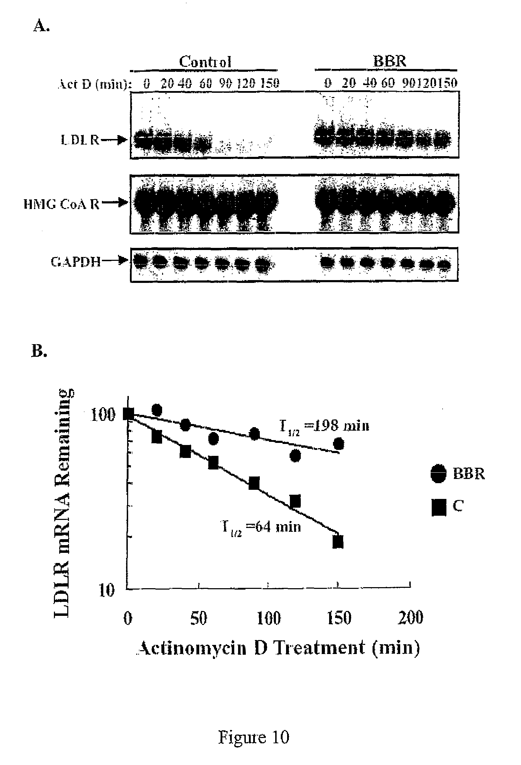

HepG2 cells were cultured and then left alone or treated with berberine for 15 hours. After 15 hours, actinomycin D (5µg/ml) was added to cells at 0, 20, 40, 60, 90, 120, or 150 minutes. Total mRNA was isolated and analyzed by Northern blot for the amount of LDLR mRNA As can be seen in Figure 10, berberine prolonged the turnover rate of LDLR transcript by approximately threefold. In contrast, the mRNA stability of HMG-CoA reductase was not altered by berberine.

### Example IX

### Transfection of HepG2 cells

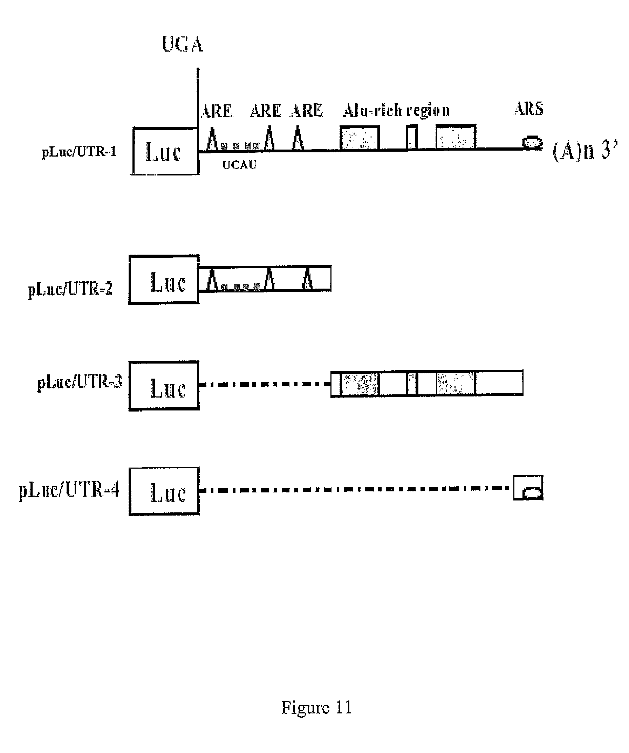

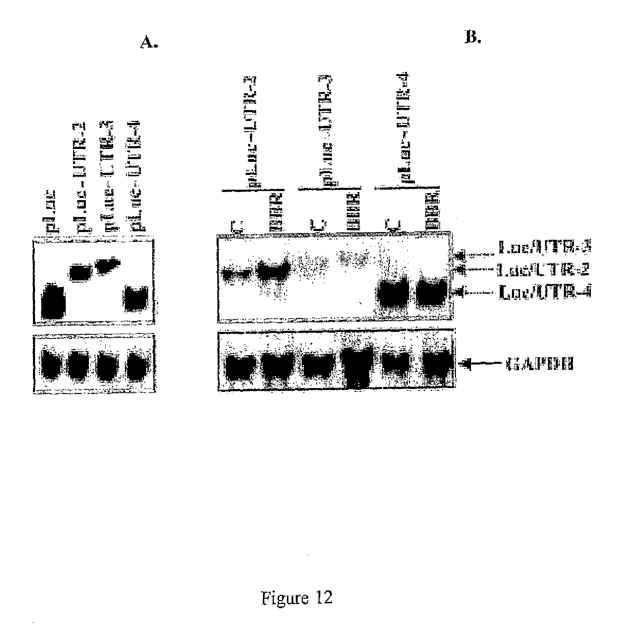

Three consecutive fragments of LDLR 3'UTR were inserted into a cytomegalovirus promoter driven *Luc* plasmid (pLuc) at the 3' end of the Luc coding sequence before the SV40 polyadenylation signal. The wild-type *Luc* reporter plasmid pLuc was constructed by insertion of the *Luc* cDNA into the *Hind*III and Xba sites of pcDNA3.1/Zeo(+). Addition of the *LDLR* 3/UTR was accomplished by PCR amplifying different regions of the 2 5kb 3'UTR of *LDLR* mRNA using XbaI-tailed primers and pLDLR3 as the template. The wild type pLuc and the chimeric plasmids pLuc-UTR-2, UTR-3 and UTR-4 were transfected into HepG2 cells (Figure 11). Cells seeded in culture dishes were transiently transfected with the chimeric plasmids. Twenty-four hours after transfection, cells were trypsinized and reseeded equally into two dishes for each plasmid transfection. After overnight incubation, one dish was treated with dimethylsulfoxide as the solvent control and another was treated with berberine for eight hours. To detect the presence of *Luc-LDLR* fusion transcripts, a PCR reaction was performed to amplify a 550 base pair fragment of *Luc* coding region with 5' primer *Luc-2up* (5'-GCTGGAGAGCAACTGCARAAGGC-3') (SEQ ID NO:1) and the 3' primer *Luc*-2lo (5'-GCAGACGAGTAGATCCAGAGG-3') (SEQ ID NO:2) using pGL3-basic as the template The PCR fragment was labeled with ³²P and used in the northern blot analysis to measure expression of *Luc* mRNA and *Luc-*LDLR 3'UTR chimeric fusion. As can be seen in figure 12, inclusion of UTR-2 and UTR-3 sequences reduced expression levels of'Luc MRNA by approximately 3-4 fold, indicating the presence of destabilization determinants within these groups whereas the *Luc* mRNA levels were only moderately reduced by fusing with UTR-4. Berberine increased the level of Luc-UTR-2 mRNA by 2.5 fold without affecting expressions of LucUIR-3 and Luc-UTR-4 or the wild type. This demonstrates that berberine affected the mRNA stability of the heterologous *Luc*-LDLR transcript and that the stabilization is mediated through regulatory sequences present in the 5' proximal region of the LDLR 3'UTR (nt 2677-3582)

### Example X

### Determination of the Role_of ARE and UCAU motif in Berberine mediated LDLR mRNA stabilization

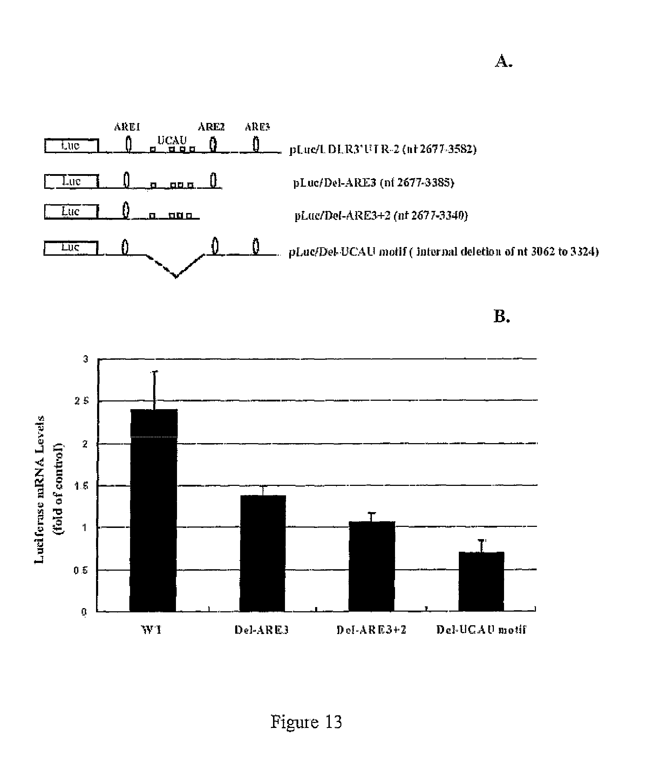

To create ARE deletion constructs, an Apa site at nt 3,384 was generated for deleting ARE3, and an Apa1 site at nt 3,334 for deleting ARE2 by site-directed mutagenesis using pLuc/UTR-2 as the template. Mutated plasmids were cut with Apa1 to remove the ARE-containing region and then the remaining vector was religated with the 5' proximal region of UTR-2. To create the UCAU motif deletion, two SacII sites for internal deletion of nt 3.062-3,324 were generated using UTR-2 as the template. All constructs were sequenced an the correct clones were further propagated to isolate plasmid DNA. These constructs and the berberine responsive wild-type construct were transfected into HepG2 cells. The effects of berberine on the chimeric *Luc* transcripts were determined by measuring *Luc* mRNA using a quantitative real-time RT-PCR assay Deletion of the ARE3 region resulted in a partial loss of berberine stimulation and deletion of'both the ARE3 and ARE2 rendered the construct unresponsive to berberine. The stabilizing effect of berberine on the *Luc* transcript was also abolished by deleting the UCAU motifs. (Figure 13).

### Example XI

### Activation of the MEK1-ERK pathway by berberine

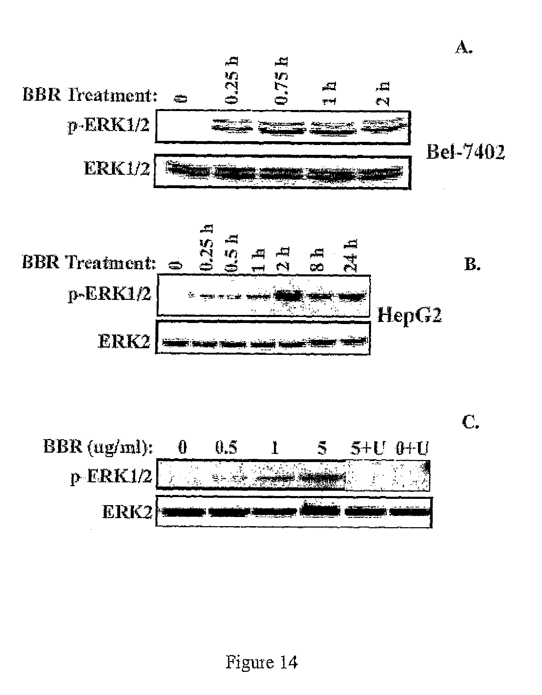

HepG2 or Bel-7402 cells were treated with berberine for 0.25, 0.5, 0.75, 1, 2, 8, and 24 hours respectively and tested for levels of' activated ERK by western blotting using antibodies that only recognize the activated (phosphorylated) ERK. In both hepatoma cell lines, berberine rapidly activated ERK and the kinetics of ERK activation preceded the upregulation of LDLR expression by berberine (Figure 14A and B) The activation of berberine is also dose dependent (Figure 14C). These data indicate that activation of ERK pathway is a prerequisite event in the berberine mediated stabilization of the LDLR transcript.

### Example XII

### Pharmacokinetics of Berberine

Healthy human volunteers were given 300 mg of berberine orally Blood samples were taken 0.5, 1, 2, 3, 4, 5, 7, 12 and 24 hours after administration and evaluated for berberine concentration by HPLC. The blood concentration curve was analyzed by 3P87. Pharmacokinetics Software program (Chinese Pharmacological Association, China). Using a one compartment model, the median pharmacokinetic parameter estimates (ranges) were as follows: Peak Time: Tpeaking: 237 hr, peak concentration: Cmax: 394.7ηg/ml, vanishing half life: T1/2: 2.91 h, the area under the curve AUC: 2799.0µg/L h, clear rate CL: 130 5L/h. The average drug retention time was 32.63 hours.

In parallel animal model studies, four canine (beagle) subjects were given 45 mg/kg of berberine orally. Serum concentrations of the drug were determined by HPLC at 2 and 3 hours after administration. There was no obvious spectrum peak detected suggesting that the concentration was below the minimum detection limit of 10 ηg/ml. One dog receiving 280mg/kg of berberine had a berberine peak showing a concentration of 31.4 ηg/ml after two hours and 22 6 ηg three hours after administration. After the berberine had cleared the system, the same dog was then administered 700 mg/kg of berberine and blood samples were taken 2, 3, 5, 7, 9 and 24 hours after administration resulting in concentrations of 21.51, 44.89, 49.54, 36.35, 27.83, and 16.01 ηg/ml respectively.

Four beagles were injected intravenously with 100 mg/kg of berberine. Using a two compartment model, the pharmacokinetic parameter estimates were as follows: Vanishing half life I1/2B is 12 59±8.83h. Area under the curve AUC is 1979.31±1140.31µg/h L; blood clearance rate: CL is 60 70±24.38L/h.

In additional, parallel animal model studies, 3H-berberine was administered intravenously to 5 rabbits (25MBq/kg) and through intravenous drip to four rabbits (46.25Mbq/kg), 0.1ml of'blood was removed at various times and radiation emissions were measured. The pharmacokinetic parameter estimates for both groups were as follows: T1/2α respectively: 1.41±0.16h, 1.03±0.11h, and I1/2β respectively: 35.3±1.3h, 35.8±2 0h, Vd respectively 20±3L/kg and 22.1±1.7L/kg.

50mg/kg of berberine was administered through stomach infusion to six rabbits Serum samples were taken at various points after administration and RP-HPLC was used to measure the drug concentration. The blood drug concentration time data was analyzed using the 3P87 pharmacokinetics software program (Chinese Pharmacological Association, China). Using automated fitting, the rabbit berberine pharmacokinetics model was found to match with the One Compartment Open model. The main pharmacokinetics parameters were as follows: peak time Tpeak: 0.63±0.25h, peak concentration Cmax: 92.72±50.89ηg/ml, vanishing half life: T1/2β: 3.11±0.58h, the area under the curve, AUC:491.7±295.5µg h/L. The results indicate that berberine can be absorbed rapidly to reach the effective concentration.

The rabbit blood protein binding rate was measured by *in vitro* dialysis at a rate of 38±3% (XD±S, n=6).

In yet additional animal models studies, mice were injected in the tail vein with 3H-berberine (135LBq/10g). Tissue radiation emission was measured 5 minutes to 2 hours after administration with the distribution of the berberine concentrations from highest to lowest being: lung>liver>kidney>spleen> heart>intestine>stomach>brain.

In a final series of animal model studies, rats were orally administered 3H-bereberine. Forty-eight hours after administration, excretions were tested for the presence of berberine 2 7% of the oral dose was measured in the urine and 86% of the oral dose was measured in the fecal matter.

Rats received intravenous berberine (9.25MBq/kg) Six days accumulation of rat urine and fecal secretions were measured for the presence of berberine. 73% of the intravenous dose of the berberine was found in the accumulated urine in both metabolized and unmetabolized forms. 10.9% of the intravenous dose was found in the fecal matter.

Three rats were given berberine (9.25MBq/kg) intravenously After 24 hours, gall bladder secretions were collected and evaluated for the presence of berberine. There was 10 1±0.9% (x±SD, n=3) of berberine in the gall bladder secretions.

### Example XIII

### Toxicity analysis of berberine

Rats and mice were administered berberine through a variety of techniques, including orally, through subcutaneous injection, peritoneal injection and intravenous injection

In rats, toxicity was achieved with an oral dose of LD₅₀>15000mg/kg. Toxicity through subcutaneous injection was LD₅₀ 7970-10690mg/kg. Toxicity through peritoneal injection was LD₅₀=138.1-146.2mg/kg and LD₅₀ 46.2-63.3 mg/kg when the berberine was administered through intravenous injection.

In mice, toxicity was achieved with an oral dose of LD₅₀> 29586-4500 mg/kg Toxicity through subcutaneous injection was LD₅₀ 139-20 mg/kg. Toxicity through peritoneal injection was LD₅₀ 30-32 2 mg/kg and LD₅₀ 7.6-10.2 mg/kg with intravenous injection.

For long term toxicity determination, rats were administered 300mg/kg of berberine orally for 182 days No abnormalities were found in blood tests, blood biochemistry, urine analysis or histopathology.

To assess teratologic potential, pregnant mice were orally administered a daily dose of between 30.480 mg/kg of berberine beginning on day 7 of the pregnancy and continuing for seven days. Rats were administered berberine beginning on day 9 of their pregnancy for seven days No birth defects were evident.

### Example XIV

### Exemplary Combinatorial Therapy Employing Berberine and Lovastatin

In accordance with the above teachings, combinatorial drug therapy employing a berberine compound or berberine related or derivative compound of Formula I, in combination with an exemplary, secondary anti-hyperlipidemia agent, was demonstrated using berberine and an exemplary statin, lovastatin, in rat model subjects. The proceedures for this study accord with those of the foregoing example, and the results are provided in Table 6, below.

**Table 6. Combinatoral anti-hyperlipidemia efficacy of berberine and lovastatin in a coordinate treatment regimen Cholesterol and LDL concentration in mmol/L**

| Treatment | Cholesterol Day 0 | Cholesterol Day 15 | LDL Day 0 | LDL Day 15 | Number of rats | P value |
|---|---|---|---|---|---|---|
| Normal diet | 1.35 | 1.30 | 0.8 | 0.85 | 5 | |
| High fat diet | 3.6 | 3.5 | 2.2 | 2.1 | 9 | |
| High fat diet and Berberine (80mg/kg/day) | 365 | 275 | 2 25 | 1.7 | 9 | <0.05 |
| High fat diet and Lovastatin (10mg/kg/day) | 3.6 | 27 | 2 15 | 1 62 | 9 | <0.05 |
| High fat diet and Berberine+lovastatin | 3 8 | 2 6 | 2 3 | 155 | 11 | <0 01 |

| | | | | | | |
|---|---|---|---|---|---|---|
| Day 0 represents: untreated rats Day 15 represents: rats treated for 15 days | | | | | | |

The foregoing data evince combinatorial effectiveness of an exemplary berberine compound employed in a coordinate treatment protocol with a secondary anti-hyperlipidemia agent, in accordance with the teachings herein above.

Although the foregoing invention has been described in detail by way of example for purposes of clarity of understanding, it will be apparent to the artisan that certain changes and modifications may be practiced within the scope of the appended claims which are presented by way of illustration not limitation in this context, various publications and other references have been cited within the foregoing disclosure for economy of description Each of these references is incorporated herein by reference in its entirety for all purposes it is noted, however, that the various publications discussed herein are incorporated solely for their disclosure prior to the filing date of the present application, and the inventors reserve the right to antedate such disclosure by virtue of prior invention.

### References

### (All disclosure and description contained in the following publications are collectively incorporated herein by reference for all purposes)

Adams CM, Goldstein JL, Brown MS. Cholesterol-induced conformational change in SCAP enhanced by Insig proteins and mimicked by cationic amphiphiles Proc Natl Acad Sci USA 100:10647-10652 (2003).
Ansell, B.I., Watson, K.E. & Fogelman, A.M. An evidence-based assessment of the NCEP adult treatment panel II guidelines. National cholesterol education program. JAMA 282, 2051-2057 (1999).
Austin MA, Hutter CM, Zimmern RL, Humphries SE. Genetic causes of monogenic heterozygous familial hypercholesterolemia: a HuGE prevalence review. Am J Epidemiol 160:407-420 (2004).
Auwerx JH, Chait A, Wolfbauer G, Deeb SS. Involvement of second messengers in regulation of the low-density lipoprotein receptor gene Mol Cell Biol 9:2298-2302 (1989).
Bakker O, Hudig F, Meijssen S, Wiersinga WM. Effects of triiodothyronine and amiodarone on the promoter of the human LDL receptor gene. Biochem Biophys Res Commun 249:517-521 (1998).
Basheeruddin K, Li X, Rechtoris C, Mazzone I Platelet-derived growth factor enhances Sp1 binding to the LDL receptor gene. Arterioscler Thromb Vasc Biol 15:1248-1254 (1995).
Bays H, Stein EA. Pharmacotherapy for dyslipidaemia - current therapies and future agents. Expert Opin Pharmacother 4:1901-1938 (2003).
Bensch, W.R., Gadski, R.A, Bean, J.S., et al., Effects of LY295427, a low-density lipoprotein (LDL) receptor up-regulator, on LDL receptor gene transcription and cholesterol metabolism in normal and hypercholesterolemic hamsters. J.Pharmacology & Experimental Therapeutics 289, 85-92(1999).
Briggs MR, Yokoyama C, Wang X, Brown MS, Goldstein IL Nuclear protein that binds sterol regulatory element of low density lipoprotein receptor promoter I. Identification of the protein and delineation of its target nucleotide sequence. J Biol Chem 268:14490-14496 (1993).
Brown AJ, Sun L, Feramisco JD, Brown MS, Goldstein JL. Cholesterol addition to ER membrane alters conformation of SCAP, the SREBP escort protein that regulates cholesterol metabolism. Mol Cell 10:237-245 (2002).
Brown MS, Goldstein JL Receptor-mediated pathway for cholesterol homeostasis. Science 232:34-47 (1986).
Brown MS, Goldstein JL. A proteolytic pathway that controls the cholesterol content of membranes, cells, and blood Proc Natl Acad Sci USA 96:11041-11048 (1999).
Brüning JC, Lingohr P, Gillette J, Hanstein B, Avci H, Krone W, Müller-Wieland D, Kotzka J Estrogen receptor-α and Sp 1 interact in the induction of the low density lipoprotein-receptor J Steroid Biochem Mol Biol 86:113-121 (2003).
Catapano. The low density lipoprotein receptor: structure, function and pharmacological modulation. Pharmac Ther 43:187-219 (1989).
Chang R, Yang E, Chamblis D, Kumar A, Wise J, Mehta KD. In vivo role of the Sp1 site neighboring sterol-responsive element-1 in controlling low-density lipoprotein receptor gene expression Biochem Biophys Res Commun 218:733-739 (1996).
Cho, E Berberini Hydrochloride, In: Pharmacopoeia of the People's Republic of China Pharmacopoeia of the People's Republic of China 2, 437-439 (1990).
Corsini A, Bellosta S, Baetta R, Fumagalli R, Paoletti R, Bernini F New insights into the pharmacodynamic and pharmacokinetic properties of statins. Pharmacol Iher 84:413-428 (1999)
Davis CG, Van Driel IR, Russell DW, Brown MS, Goldstein JL. The LDL-receptor: identification of aminoacids in cytoplasmic domain required for rapid endocytosis. J Biol Chem 262:4075-4079 (1987).
Defesche JC. Low-density lipoprotein receptor-its structure, function, and mutations Semin Vasc Med 4:5-11 (2004).
Dixon, D.A., Kaplan, C.D, McIntyre, T.M., Zimmerman, G.A., Prescott, S.M. Post-transcriptional control of cyclooxygenase gene expression J.Biol Chem. 275, 11750-11757 (2000).
Duncan EA, Brown MS, Goldstein JL, Sakai J. Cleavage site for sterol-regulated protease located to a Leu-Ser bond in the lumenal loop of sterol regulatory element-binding protein-2. J Biol Chem 272:12778-12785 (1997).
Duncan EA, Dave UP, Sakai J, Goldstein JL, Brown MS Second-site cleavage in sterol regulatory element-binding protein occurs at transmembrane junction as determined by cysteine panning. J Biol Chem 273:17801-17809 (1998).
Duntas LH. Thyroid disease and lipids, Thyroid 12:287-293 (2002).
Duvillard L, Florentin E, Lizard G, Petit JM, Galland F, Monier S, Gambert P, Verges B Cell surface expression of LDL receptor is decreased in type 2 diabetic patients and is normalized by insulin therapy Diabetes Care 26:1540-1544 (2003).
Espenshade PJ, Li WP, Yabe D. Sterols block binding of COPII proteins to SCAP, thereby controlling SCAP sorting in ER Proc Natl Acad Sci USA 99:11694-11699 (2002).
Expert panel on detection evaluation and treatment of high blood cholesterol in adults. Executive summary of the third report of the national cholesterol education program (NCEP). JAMA 285, 2486-2497 (2001).
Gierens H, Nauck M, Roth M, Schinker R, Schürmann C, Scharnagl H, Neuhaus G, Wieland H, März W Interleukin-6 stimulates LDL receptor gene expression via activation of sterol-responsive and Sp1 binding elements. Arterioscler Thromb Vasc Biol 20:1777-1783 (2000).
Goldstein, J.L. & Brown, M.S Regulation of the mevalonate pathway Nature 343, 425-430 (1990).
Goldstein, J L, Rawson, R.B. & Brown, M.S. Mutant mammalian cells as tools to delineate the sterol regulatory element-binding protein pathway for feedback regulation of lipid synthesis. Archives of Biochemistry and Biophysics 397, 139-148 (2002).
Goto D, Okimoto T, Ono M, Shimotsu H, Abe K, Tsujita Y, Kuwano M (1997) Upregulation of low density lipoprotein receptor by gemfibrozil, a hypolipidemic agent, in human hepatoma cells through stabilization of mRNA transcripts Arterioscler Thromb Vasc Biol 17:2707-2712.
Graham A, Russell L.J. Stimulation of low-density lipoprotein uptake in HepG2 cells by epidermal growth factor via a tyrosine kinase-dependent, but protein kianse C-independent mechanism Biochem J 298:579-584 (1994).
Grand-Perret, T., Bouillot, A., Perrot, A , Commans, S., Walker, M. & Issandou, M. SCAP ligands are potent new lipid-lowering drugs, Nature Medicine 7, 1332-1338 (2001).
Grundy, S.M. Statin trials and goals of cholesterol-lowering therapy. Circulation 97, 1436-1439 (1998).
Hardardottir I, Grunfeld C, Feingold KR Effects of endotoxin and cytokines on lipid metabolism. Curr Opin Lipidol 5:207-215 (1994).
Hirano Y, Yoshida M, Shimizu M, Sato R Direct demonstration of rapid degradation of nuclear sterol regulatory element-binding proteins by the ubiquitin-proteasome pathway J Biol Chem 276:36431-36437 (2001).
Hobbs HH, Russell DW, Brown MS, Goldstein JL The LDL receptor locus in familial hypercholesterolemia: mutation analysis of a membrane protein. Annu Rev Genet 24:133-170 (1990).
Hodgin JB, Maeda N. Minireview: estrogen and mouse models of atherosclerosis Endocrinology 143:4495-4501 (2002).
Horton JD, Goldstein JL, Brown MS. SREBPs: activators of the complete program of cholesterol and fatty acid synthesis in the liver. J Clin Invest 109:1125-1131 (2002).
Horton, J.D., Cuthbert, J.A. & Spady, D.K. Dietary fatty-acids regulate hepatic low-density lipoprotein (LDL) transport by altering LDL receptor protein and messenger-RNA levels. J.Clinc.Invest 92, 743-749 (1993).
Hsu HY, Nicholson AC, Hajjar DP. Basic fibroblast growth factor-induced low density lipoprotein receptor transcription and surface expression Signal transduction pathways mediated by the bFGF receptor tyrosine kinase. J Biol Chem 269:9213-9220 (1994).
Hua X, Nohturfft A, Goldstein JL, Brown MS. Sterol resistance in CHO cells traced to point mutations in SREBP cleavage activating protein (SCAP). Cell 87:415-426 (1996).
Hua X, Sakai J, Ho YK, Goldstein JL, Brown MS Hairpin orientation of sterol regulatory element-binding protein-2 in cell membranes as determined by protease protection J Biol Chem 270:29422-2942 (1995).
Huang W, Mishra V, Batra S, Dillon I, Mehta KD Phorbol ester promotes histone H3-Ser10 phosphorylation at the LDL receptor promoter in a protein kinase C-dependent manner J Lipid Res 45:1519-1527 (2004).
Inukai T, Takanashi K, Takebayashi K, Tayama K, Aso Y, Takiguchi Y, Takemura Y. Estrogen markedly increases LDL-receptor activity in hypercholesterolemic patients J Med 31:247-261 (2000).
Kapoor GS, Atkins BA, Mehta KD Activation of Raf-1/MEK-1/2/p42/44MAPK cascade alone is sufficient to uncouple LDL receptor expression from cell growth Mol Cell Biochem 236:13-22 (2002).
Kapoor GS, Golden C, Atkins B, Mehta KD. pp90RSK- and protein kinase C-dependent pathway regulates p42/44MAPK-induced LDL receptor transcription in HepG2 cells. J Lipid Res 44:584-593 (2003).
Knouff C, Malloy S, Wilder J, Altenburg MK, Maeda N Doubling expression of the low density lipoprotein receptor by truncation of the 3' -untranslated region sequence ameliorates type III hyperlipoproteinemia in mice expressing the human ApoE2 isoform J Biol Chem 276:3856-3862 (2001).
Kong W, Abidi P, Kraemer FB, Jiang JD, Liu J In vivo activities of cytokine oncostatin M in regulation of plasma lipid levels J Lipid Res 46:1163-1171 (2005).
Kong W, Wei J, Abidi P, Lin M, Inaba S, Li C, Wang Y, Wang Z, Si S, Pan H, Wang S, Wu J, Wang Y, Li Z, Liu J, Jiang JD. Berberine is a novel cholesterol-lowering drug working through a unique mechanism distinct from statins Nat Med 10:1344-1351 (2004)
Kotzka J, Lehr S, Roth G, Avci H, Knebel B, Müller - Wieland D. Insulin-activated Erk-mitogen-activated protein kinases phosphorylate sterol regulatory element-binding protein-2 at serine residues 432 and 455 in vivo J Biol Chem 279:22404-22411 (2004).
Kotzka J, Müller-Wieland D, Koponen A, Njamen D, Kremer L, Roth G, Munck M, Knebel B, Krone W. ADD1/SREBP-1c mediates insulin-induced gene expression linked to the MAP kinase pathway Biochem Biophys Res Commun 249:375-379 (1998).
Kotzka J, Müller-Wieland D, Roth G, Kremer L, Munck M, Schürmann S, Knebel B, Krone W Sterol regulatory element binding proteins (SREBP)-1a and SREBP-2 are linked to the MAP kinase cascade. J Lipid Res 41:99-108 (2000).
Kumar A, Middleton A, Chambers TC, Mehta KD Differential roles of extracellular signal-regulated kinase-1/2 and p38MAPK in interleukin-1β- and tumor necrosis factox-α-induced low density lipoprotein receptor expression in HepG2 cells. J Biol Chem 273:15742-15748 (1998).
LaRosa, J.C- & He, J.V.S. Effect of'statins on risk of coronary disease: a meta-analysis of randomized controlled trials JAMA 282, 2340-2346 (1999).
Lau,C.W, Yao, X.Q., Chen, Z Y., Ko, W H., Huang, Y. Cardiovascular actions of berberine Cardiovasc Drug Rev. 19, 234-244 (2001).
Li C, Briggs MR, Ahlborn TE, Kraemer FB, Liu J. Requirement of Sp1 and estrogen receptor α interaction in 17β-estradiol-mediated transcriptional activation of the low density lipoprotein receptor gene expression Endocrinology 142:1546-1553 (2001).
Li C, Kraemer FB, Ahlborn TE, Liu J. Induction of low density lipoprotein receptor (LDLR) transcription by oncostatin M is mediated by the extracellular signal-regulated kinase signaling pathway and the repeat 3 element of the LDLR promoter. J Biol Chem 274:6747-6753 (1999).
Lindgren V, Luskey KL, Russell DW, Francke U. Human genes involved in cholesterol metabolism: chromosomal mapping of the loci for the low-density lipoprotein receptor and 3-hydroxy-3-methylglutaryl-coenzyme A reductase with cDNA probe. Proc Natl Acad Sci USA 82:8567-8571 (1985).
Liu J, Ahlborn TE, Briggs MR, Kraemer FB. Identification of a novel sterol-independent regulatory element in the human low density lipoprotein receptor promoter. J Biol Chem 275:5214-5221 (2000).
Liu J, Streiff R, Zhang YL, Vestal RE, Spence MJ, Briggs MR. Novel mechanism of transcriptional activation of hepatic LDL receptor by oncostatin M. J Lipid Res 38:2035-2048 (1997).
Liu, J., Zhang, F., Li, C., Lin, M. and Briggs, M R. Synergistic activation of human LDL receptor expression by SCAP ligand and cytokine oncostatin M. Arterioscler.Throm.Vasc.Biol 23:90-96, (2003).
Liu, Berberine. Altern Med Rev. 5, 175-177 (2000).
Luo, L.J. Experience of berberine in the treatment of diarrhea. Chin.J.Med. 41, 452-455 (1955). Mazzone T, Basheeruddin K, Ping L, Frazer S, Getz GS. Mechanism of the growth-related activation of the low density lipoprotein receptor pathway. J Biol Chem 264:1787-1792 (1989).
Mehta KD. Role of mitogen-activated protein kinases and protein kinase C in regulating low-density lipoprotein receptor expression Gene Expr 10:153-164 (2002).
MRC/BHF. Heart protection study of cholesterol lowering with simvastatin in 20536 high-risk individuals: a randomised placebo-controlled trial Lancet 360, 7-22 (2002).
Nakahara M, Fujii H, Maloney PR, Shimizu M, Sato R Bile acids enhance low density lipoprotein receptor gene expression via a MAPK cascade-mediated stabilization of mRNA J Biol Chem 277:37229-37234 (2002).
Ness GC. Thyroid hormone Basis for its hypocholesterolemic effect. J Fla Med Assoc 78:383-385 (1991).
Ness GC, Lopez D. Transcriptional regulation of rat hepatic low-density lipoprotein receptor and cholesterol 7-α-hydroxylase by thyroid hormone. Arch Biochem Biophys 323:404-408 (1995).
Nohturfft A, Brown MS, Goldstein JL. Topology of SREBP cleavage activating protein, a polytopic membrane protein with a sterol sensing domain. J Biol Chem 273:17243-17250 (1998).
Nohturfft A, DeBose-Boyd RA, Scheek S, Goldstein JL, Brown MS Sterols regulate cycling of SREBP cleavage-activating protein (SCAP) between endoplasmic reticulum and Golgi Proc Natl Acad Sci USA 96:11235-11240 (1999).
Norturfft, A , DeBose-Boyd, R.A., Scheek, S., Goldstein, J L. & Brown, M.S. Sterols regulate cycling of SREBP cleavage-activating protein (SCAP) between endoplasmic reticulum and Golgi Proc.Natl.Accad.Sci USA 96, 11235-11240 (1999).
Parini P, Angelin B, Rudling M Importance of estrogen receptors in hepatic LDL receptor regulation. Arterioscler Thromb Vasc Biol 17:1800-1805 (1997).
Radhakrishnan A, Sun LP, Kwon HJ, Brown MS, Goldstein JL. Direct binding of cholesterol to the purified membrane region of SCAP: mechanism for a sterol-sensing domain Mol Cell 15:259-268 (2004).
Rawson RB The SREBP pathway - insights from Insigs and insects Nat Rev Mol Cell Biol 4:631-640 (2003).
Robinson MJ, Cobb MH Mitogen-activated protein kinase pathways Curr Opin Cell Biol 9:180-186 (1997).
Roth G, Kotzka J, Kremer L, Lehr S, Lohaus C, Meyer HE, Krone W, Müller-Wieland D MAP kinases Erk1/2 phosphorylate sterol regulatory element-binding protein (SREBP)-1a at serine 117 in vitro. J Biol Chem 275:33302-33307 (2000).
Sakai, J. & Rawson, R.B. The sterol regulatory element-protein pathway: control of lipid homeostasis through regulated intracellular transport Curr Opin Lipidol 12, 261-266 (2001).
Singh RP, Dhawan P, Golden C, Kapoor GS, Mehta KD. One-way cross-talk between p38MAPK and p42/44MAPK Inhibition of p38MAPK induces low density lipoprotein receptor expression through activation of p42/44MAPK cascade J Biol Chem 274:19593-19600 (1999).
Smith JR, Osborne TF, Goldstein JL, Brown MS Identification of nucleotides responsible for enhancer activity of sterol regulatory element in low density lipoprotein receptor gene. J Biol Chem 265:2306-2310 (1990).
Soutar AK. Intracellular transport of the low-density lipoprotein receptor Biochem Soc Trans 24:547-552 (1996).
Soutar AK, Knight BL. Structure and regulation of the LDL-receptor and its gene. Br Med Bull 46:891-916 (1990).
Spady DK, Hepatic clearance of plasma low-density lipoproteins. Semin Liver Dis 12:373-385 (1992).
Stopeck AI, Nicholson AC, Mancini FP, Hajjar DP. Cytokine regulation of low density lipoprotein receptor gene transcription in HepG2 cells. J Biol Chem 268:17489-17494 (1993).
Streicher R, Kotzka J, Müller-Wieland D, Siemeister G, Munck M, Avci H, Krone W. SRBBP-1 mediates activation of the low density lipoprotein receptor promoter by insulin and insulin-like growth factor-I J Biol Chem 271:7128-7133 (1996).
Südhoff TC, Goldstein JL, Brown MS, Russell DW The LDL-receptor gene A mosaic of exons shared with different proteins Science 228:815-822 (1985).
Sudhoff TC, Van Der Westhuyzen DR, Goldstein JL, Brown MS, Russell DW. Three direct repeats and a TATA-like sequence are required for regulated expression of the human low density lipoprotein receptor gene J Biol Chem 262:10773-10779 (1987).
Sugiura, R, Kita, A, Shimizu, Y., Shuntoh, H., Sio, S.O. & Kuno, T Feedback regulation of MAPK signaling by an RNA-binding protein. Nature 424, 961-965 (2003).
Tolleshaug H, Goldstein JL, Schneider W.J, Brown MS. Posttranslational processing of the LDL receptor and its genetic disruption in familial hypercholesterolemia Cell 30:715-724 (1982).
Ugawa, T, Kakuta, H, Moritani, H. and Inagaki, O. Effect of YM-53601, a novel squalene synthase inhibitor, on the clearance rate of plasma LDL and VLDL in hamsters. British J of Pharmacology 137, 561-567 (2002).
Liu, J., Hadjokas, N., Mosley, B., Vestal, R.E. Oncostatin M-specific receptor expression and function in regulating cell proliferation of normal and malignant mammary epithelial cells. Cytokine 10, 295-302 (1998).
Wade DP, Knight BL, Soutar AK Regulation of low-density-lipoprotein-receptor mRNA by insulin in human hepatoma HepG2 cells Eur J Biochem 181:727-731.
Wang X, Briggs MR, Hua X, Yokoyama C, Goldstein JL, Brown MS (1993) Nuclear protein that binds sterol regulatory element of low density lipoprotein receptor promoter II. Purification and characterization J Biol Chem 268:14497·14504.
Wang X, Sato R, Brown MS, Hua X, Goldstein JL (1994) SREBP-1, a membrane-bound transcription factor released by sterol-regulated proteolysis Cell 77:53-62 (1989).
Wilson GM, Roberts EA, Deeley RG Modulation of LDL receptor mRNA stability by phorbol esters in human liver cell culture models J Lipid Res 38:437-446 (1997).
Wilson GM, Vasa MZ, Deeley RG. Stabilization and cytoskeletal-association of LDL receptor mRNA are mediated by distinct domains in its 3' untranslated region J Lipid Res 39:1025-1032 (1998).
Windler EET, Kovanen PI, Chao Y, Brown MS, Havel RJ, Goldstein JL. The estradiol-stimulated lipoprotein receptor of rat liver J Biol Chem 255:10464-10471 (1980).
Yabe D, Brown MS, Goldstein JL Insig-2, a second endoplasmic reticulum protein that binds SCAP and blocks export of sterol regulatory element-binding proteins. Proc Natl Acad Sci USA 99:12753-12758 (2002).
Yamamoto I, Davis CG, Brown MS, Schneider WJ, Casey ML, Goldstein JL, Russell DW. The human LDL receptor: a cysteine-rich protein with multiple Alu sequences in its mRNA. Cell 39:27-38 (1984).
Yang T, Espenshade PJ, Wright ME, Yabe D, Gong Y, Aebersold R, Goldstein JL, Brown MS. Crucial step in cholesterol homeostasis: sterols promote binding of SCAP to Insig-1, a membrane protein that facilitates retention of SREBPs in ER. Cell 110:489-500 (2002).
Yang, I., Goldstein, J L & Brown, M.S. Overexpression of membrane domain of SCAP prevents sterols from inhibiting SCAP SREBP exit from endoplasmic reticulum J Biol. Chem 275, 29881-29886 (2000).
Yeung, A & Isao, P Statin therapy: beyond cholesterol lowering and antiinflammatory effects. Circulation 105, 2937-2938 (2002).
Yokoyama C, Wang X, Briggs MR, Admon A, Wu J, Hua X, Goldstein JL, Brown MS. SREBP-1, a basic-helix-loop-helix-leucine zipper protein that controls transcription of the low density lipoprotein receptor gene. Cell 75:187-197 (1993).
Zhang F, Lin M, Abidi P, Thiel G, Liu J. Specific interaction of Egr1 and c/EBPβ leads to the transcriptional activation of the human low density lipoprotein receptor gene J Biol Chem 278:44246-44254 (2003).
Zhang, F., Ahlborn, I.E., Li, C., Kraemer, F.B. & Liu, J. Identification of Egr1 as the oncostatin M-induced transcription activator that binds to the sterol-independent regulatory element of the human LDL receptor promoter. J.Lipid Res. 43, 1477-1485 (2002).
Zhao, S.P. Etiology and diagnosis of hyperlipidemia. Clinical Serum and Lipid 1, 72-94 (1997). Zubiaga AM, Belasco JG, Greenberg ME. The nonamer UUAUUUAUU is the key AU-rich sequence motif that mediates mRNA degradation. Mol Cell Biol 15:2219-2230 (1995).

The present invention is described by way of the following clauses:
1. A method for preventing or treating hyperlipidemia in a mammalian subject comprising administering an anti-hyperlipidemia effective amount of a berberine compound or berberine related or derivative compound of Formula I, or a pharmaceutically-acceptable salt, isomer, enantiomer, solvate, hydrate, polymorph or prodrug thereof, to said subject 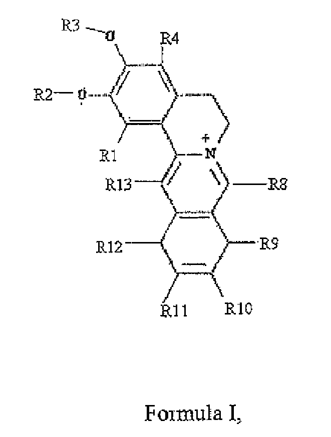 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups.
2. The method of clause 1, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxy, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxy, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
3. The method of clause 1, further comprising administering a secondary anti-hyperlipidemic agent or other adjunctive therapeutic agent that is effective in a combinatorial formulation or coordinate treatment regimen with said berberine compound or berberine related or derivative compound of Formula I to treat or prevent hyperlipidemia or another cardiovascular disease or related symptom or condition thereof in said subject.
4. The method of clause 3, wherein the secondary anti-hyperlipidemic or adjunctive therapeutic agent is administered to said subject in a coordinate administration protocol, simultaneously with, prior to, or after, administration of said berberine to the subject.
5. The method of clause 3, wherein the secondary anti-hyperlipidemic or adjunctive therapeutic agent is selected from the group consisting of: cholesterol-uptake inhibitors; cholesterol biosynthesis inhibitors, including HMG-CoA reductase inhibitors or statins; HMG-CoA synthase inhibitors; squalene epoxidase inhibitors and squalene synthetase inhibitors; acyl-coenzyme. A cholesterol acyltransferase (ACAT) inhibitors, including, melinamide; probucol; nicotinic acid and salts thereof; niacinamide; cholesterol absorption inhibitors, including, β-sitosterol and ezetimibe; bile acid sequestrant anion exchange resins, including cholestyramine, colestipol, colesevelam and dialkylaminoalkyl derivatives of a cross-linked dextran; LDL receptor inducers; fibrates, including clofibrate, bezafibrate, fenofibrate and gemfibrozil; vitamin B6 and pharmaceutically acceptable salts thereof; vitamin B12, including cyanocobalamin and hydroxocobalamin; vitamin B3; anti-oxidant vitamins, including vitamin C, vitamin E, and betacarotene; β blockers; angiotensin II receptor (AT₁) antagonists; angiotensin-converting enzyme inhibitors, renin inhibitors; platelet aggregation inhibitors, including fibrinogen receptor antagonists; hormones, including estrogen; insulin; ion exchange resins; omega-3 oils; benfluorex; ethyl icosapentate; and amlodipine
6. The method of clause 3, wherein the secondary anti-hyperlipidemic or adjunctive therapeutic agent is a statin or HMG-CoA reductase inhibitor
7. The method of clause 6, wherein the statin or HMG-CoA reductase inhibitor is lovastatin, simvastatin, pravastatin, fluvastatin, rosuvastatin, pitavastatin, or atorvastatin.
8. The method of clause 3, wherein the secondary anti-hyperlipidemic or adjunctive therapeutic agent is a cholesterol-uptake inhibitor or a cholesterol biosynthesis inhibitor.
9. The method of clause 3, wherein the secondary anti-hyperlipidemic or adjunctive therapeutic agent is an acyl-coenzyme A cholesterol acyltransferase (ACAT) inhibitor.
10. The method of clause 9, wherein the acyl-coenzyme A cholesterol acyltransferase (ACAT) inhibitor is melinamide or probucol.
11. The method of clause 3, wherein the secondary anti-hyperlipidemic or adjunctive therapeutic agent is a cholesterol absorption inhibitor.
12. The method of clause 11, wherein the cholesterol absorption inhibitor is β-sitosterol or ezetimibe.
13. The method of clause 3, wherein the secondary anti-hyperlipidemic or adjunctive therapeutic agent is an anion exchange resin
14. The method of clause 13, wherein the anion exchange resin is cholestyramine, colestipol, colesevelam or dialkylaminoalkyl derivative.
15. The method of clause 3, wherein the secondary anti-hyperlipidemic or adjunctive therapeutic agent is a fibrate.
16. The method of clause 15, wherein the fibrate is clofibrate, bezafibrate, fenofibrate or gemfibrozil.
17. The method of clause 1, further comprising advising or engaging the subject to undertake an additional therapeutic treatment selected from the group consisting of exercise, diet modification, or surgery.
18. The method of clause 3, wherein the secondary anti-hyperlipidemic or adjunctive therapeutic agent is is an herbal-derived product or extract.
19. The method of clause 18, wherein the herbal-derived product or extract is obtained or selected from the group consisting of curcumin, gugulipid, garlic, vitamin E, soy, soluble fiber, fish oil, green tea, carnitine, chromium coenzyme Q10, vitamin C, betacarotene, grape seed extract, pantothine, red yeast rice, and royal jelly.
20. A method for preventing or treating one or more symptoms of a cardiovascular disease or condition caused by hyperlipidemia in a mammalian subject comprising administering an anti-hypeilipidemic effective amount of a berberine compound or berberine related or derivative compound of Formula I, or a pharmaceutically-acceptable salt, isomer, enantiomer, solvate, hydrate, polymorph or prodrug thereof, to said subject 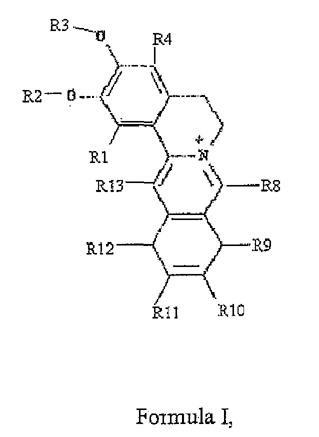 wherein each of R₁, R₂, R₃, R₄ R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups.
21. The method of clause 20, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
22. The method of clause 20, wherein said one or more symptoms of the cardiovascular disease or condition include(s) atherosclerosis, coronary artery disease, angina pectoris, carotid artery disease, stroke, cerebral arteriosclerosis, myocardial infarction, cerebral infarction, restenosis following balloon angioplasty, high blood pressure, intermittent claudication, dyslipidemia post-prandial lipidemia or xanthoma.
23. The method of clause 1, wherein said anti-hyperlipidemia effective amount comprises between about 10 to about 1500 mg of said berberine compound or berberine related or derivative compound of Formula I per day.
24. The method of clause 1, wherein said anti-hyperlipidemia effective amount comprises between about 20 mg to about 1000 mg of said berberine compound or berberine related or derivative compound of Formula I per day.
25. The method of clause 1, wherein said anti-hyperlipidemia effective amount comprises between about 25 mg to about 750 mg of said berberine compound or berberine related or derivative compound of Formula I per day.
26. The method of clause 1, wherein said anti-hyperlipidemia effective amount comprises between about 50 mg to about 500 mg of berberine per day.
27. The method of clause 1, wherein said anti-hyperlipidemia effective amount of said berberine compound or berberine related or derivative compound of Formula I is administered one, two, three, or four times per day.
28. The method of clause 1, wherein the administration of said effective amount of the berberine compound or berberine related or derivative compound of Formula I is anti-hyperlipidemia effective to decrease total cholesterol in said subject to about 200mg/dL.
29. The method of clause 1, wherein the administration of said effective amount of the berberine compound or berberine related or derivative compound of Formula I is anti-hyperlipidemia effective to decrease total cholesterol in said subject to about 175 mg/dL.
30. The method of clause 1, wherein the administration of berberine is anti-hyperlipidemia effective to decrease LDL levels in said subject to about 130 mg/dL
31. The method of clause 1, wherein the administration of'said effective amount of the berberine compound or berberine related or derivative compound of Formula I is anti-hyperlipidemia effective to decrease LDL levels in said subject by about 20mg/dL to about 50mg/dL.
32. The method of clause 1, wherein the administration of said effective amount of'the berberine compound or berberine related or derivative compound of Formula I is anti-hyperlipidemia effective to decrease triglycerides in said subject to about 150 mg/dL.
33. The method of clause 1, wherein the administration of said effective amount of the berberine compound or berberine related or derivative compound of Formula I is anti-hyperlipidemia effective to decrease triglycerides in said subject by about 20mg/dL to about 50 mg/dL.
34. The method of clause 1, wherein the administration of said effective amount of the berberine compound or berberine related or derivative compound of Formula I is anti-hyperlipidemia effective to decrease hs-CRP in said subject to about 2.0 mg/L.
35. The method of clause 1, wherein the administration of'said effective amount of the berberine compound or berberine related or derivative compound of Formula I is anti-hyperlipidemia effective to decrease hs-CRP in said subject by about 0.5mg/L to about 2.0 mg/L.
36. A method of controlling hyperlipidemia in a mammalian subject to reduce or prevent cardiovascular disease comprising administering to said subject an anti-hyperlipidemia effective amount of a berberine compound or berberine related or derivative compound of Formula I, or a pharmaceutically-acceptable salt, isomer, enantiomer, solvate, hydrate, polymorph or prodrug thereof, to said subject 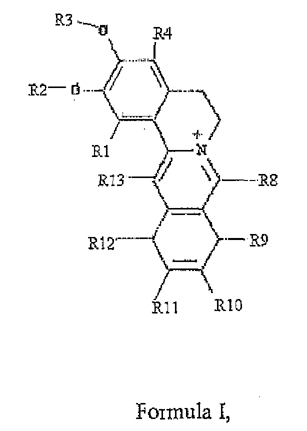 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, nifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups.
37. The method of clause 36, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxy, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br ; R₁₀ is selected from methyl, ethyl, hydroxyl, Cl, Bz; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
38. The method of clause 36, wherein the method is effective to reduce or prevent one or more symptoms, diseases, or conditions in said subject selected from atherosclerosis, coronary artery disease, angina pectoris, carotid artery disease, stroke, cerebral arteriosclerosis, high blood pressure, myocardial infarction, cerebral infarction, restenosis following balloon angioplasty, intermittent claudication, dyslipidemia post-prandial lipidemia, and xanthoma.
39. The method of clause 36, wherein the hyperlipidemia is associated with primary or secondary hyperlipidemia in said subject.
40. The method of clause 36, wherein the hyperlipidemia is associated with familial hyperchylomicronemia, familial hypercholesterolemia, familial combined hyperlipidemia, familial dysbetaliproteinemia, familial hypertriglyceridemia, familial defective apolipoprotein B-100, diabetes mellitus, hypothyroidism, uremia, nephrotic syndrome, acromegaly, obstructive liver disease, or dysproteinemia in said subject.
41. The method of clause 36, wherein the hyperlipidemia is associated with prior or current use of oral contraceptives, glucocorticoids, or antihypertensives by said subject
42. The method of clause 36, wherein the hyperlipidemia is associated with adverse dietary habits of said subject.
43. The method of clause 36, wherein the administration of said effective amount of the berberine compound or berberine related or derivative compound of Formula I is anti-hyperlipidemia effective to decrease total cholesterol in said subject to about 200mg/dL.
44. The method of clause 36, wherein the administration of said effective amount of the berberine compound or berberine related or derivative compound of Formula I is anti-hyperlipidemia effective to decrease total cholesterol in said subject to about 175 mg/dL
45. The method of clause 36, wherein the administration of said effective amount of the berberine compound or berberine related or derivative compound of Formula I is anti-hyperlipidemia effective to decrease LDL levels in said subject to about 130 mg/dL
46. The method of clause 36, wherein the administration of said effective amount of the berberine compound or berberine related or derivative compound of Formula I is anti-hyperlipidemia effective to decrease LDL levels in said subject by about 20mg/dL to about 50mg/dL.
47. The method of clause 36, wherein the administration of said effective amount of the berberine compound or berberine related or derivative compound of Formula I is anti-hyperlipidemia effective to decrease triglycerides in said subject to about 150 mg/dL.
48. The method of clause 36, wherein the administration of said effective amount of the berberine compound or berberine related or derivative compound of Formula I is anti hyperlipidemia effective to decrease triglycerides in said subject by about 20mg/dL to about 50 mg/dL.
49. The method of clause 36, wherein the administration of said effective amount of the berberine compound or berberine related or derivative compound of Formula I is anti-hyperlipidemia effective to decrease hs CRP in said subject to about 20 mg/L
50. The method of clause 36, wherein the administration of said effective amount of the berberine compound or berberine, related or derivative compound of Formula I is anti-hyperlipidemia effective to decrease hs-CRP in said subject by about 0.5mg/L to about 2.0 mg/L.
51. A method for treating one or more symptoms of cardiovascular disease comprising administering to a mammalian subject an effective amount of a berberine compound or berberine related or derivative compound of Formula I, Or a pharmaceutically-acceptable salt, isomer, enantiomer, solvate, hydrate, polymorph or prodrug thereof, to said subject 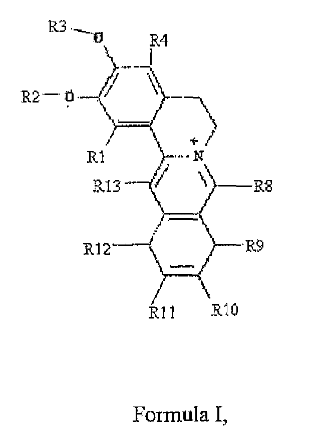 wherein each of R₁, R₂,R_{3,} R₄ R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkenyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups.
52. The method of clause 51, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1 methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1 -methyl-2ethylpiopyl.
53. The method of clause 51, wherein said one or more symptoms include(s) shortness of breath, chest pain, leg pain, tiredness, confusion, vision changes, blood in urine, nosebleeds, irregular heartbeat, loss of balance or coordination, weakness, and/or vertigo,
54. A composition for preventing or alleviating hyperlipidemia in a mammalian subject comprising an anti-hyperlipidemia effective amount of a berberine compound for berberine related or derivative compound of Formula 1, or a pharmaceutically-acceptable salt, isomer, enantiomer, solvate, hydrate, polymorph or prodrug thereof 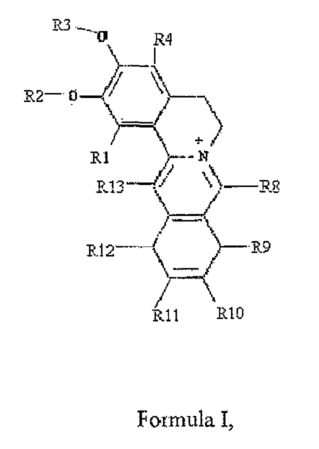 wherein each of R₁, R₂, R₃, R₄, R₅, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, alkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups.
55. The composition of clause 54, wherein R₁ is selected from methyl, ethyl, hydroxy, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C₆) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1'dimethylpiopyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2-ethylpropyl; R₉ is selected from methyl, ethyl, hydroxyl, Cl, Br; R₁₀ is selected from methyl, ethyl, hydroxyl, Cl, Bi; R₁₁ is selected from methyl, ethyl, hydroxyl, Cl, Br; R₁₂ is selected from methyl, ethyl, hydroxyl, Cl, Br; and R₁₃ is selected from straight or blanched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
56. The composition of clause 55, wherein the berberine compound or berberine related or derivative compound of Formula I is berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetrahydroberberinium iodide, 6-protobererine, 9-ethoxycarbonyl berberine, 9-N,N-dimethylcarbamoyl berberine and 12-bromo berberine, berberine azide, or berberine betaine
57. A composition for treating or preventing hyperlipidemia in a mammalian subject comprising an anti-hypeilipldemia effective amount of berberine compound or berberine related or derivative compound of Formula I, or a pharmaceutically-acceptable salt, isomer, enantiomer, solvate, hydrate, polymorph or prodrug thereof 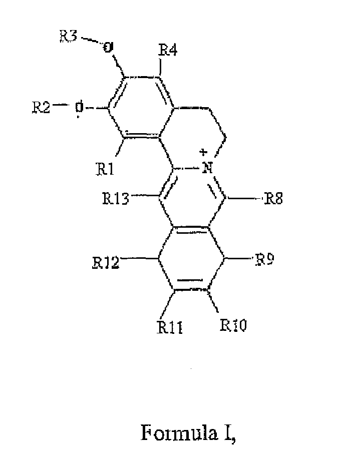 wherein each of R₁, R₂ R₃ R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups;
   and a secondary anti-hyperlipidemic agent or other adjunctive therapeutic agent useful in the treatment of a a cardiovascular disease.
58. The composition of clause 57, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or blanched (C1-C6) alkyl, including substitution selected from methyl, ethyl, n-propyl, 1-methylethyl, n-butyl, 1-methylpropyl, 2-methylpropyl, 1,1 - dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl
59. The composition of clause 57, wherein the secondary anti-hyperlipidemic or adjunctive therapeutic agent is selected from the group consisting of: cholesterol-uptake inhibitors; cholesterol biosynthesis inhibitors, including HMG-CoA reductase inhibitors or statins; HMG CoA synthase inhibitors; squalene epoxidase inhibitors and squalene synthetase inhibitors; acyl-coenzyme A cholesterol acyltransferase (ACAT) inhibitors, including, melinamide; probucol; nicotinic acid and salts thereof; niacinamide; cholesterol absorption inhibitors, including, β-sitosterol and ezetimibe; bile acid sequestrant anion exchange resins, including cholestyramine, colestipol, colesevelam and dialkylaminoalkyl derivatives of a cross-linked dextran; LDL receptor inducers; fibrates, including clofibrate, bezafibrate, fenofibrate and gemfibrozil; vitamin B6 and pharmaceutically acceptable salts thereof; vitamin B12, including cyanocobalamin and hydroxocobalamin; vitamin B3; anti-axidant vitamins, including vitamin C, vitamin E, and betacarotene; β blockers; angiotensin II receptor (AT₁) antagonists; angiotensin-converting enzyme inhibitors, renin inhibitors; platelet aggregation inhibitors, including fibrinogen receptor antagonists; hormone, including estrogen; insulin; ion exchange resins; omega-3 oils; benfluorex; ethyl icosapentate; and amlodipine.
60. The composition of clause 57, wherein the secondary anti-hyperlipidemic or adjunctive therapeutic agent is a statin or HMG-CoA reductase inhibitor.
61. The composition of clause 60, wherein the statin or HMG- CoA reductase inhibitor is lovastatin, simvastatin, pravastatin, fluvastatin, rosuvastatin, pitavastatin, or atorvastatin.
62. The composition of clause 57, wherein the secondary anti-hyperlipidemic or adjunctive therapeutic agent is a cholesterol-uptake inhibitor or a cholesterol biosynthesis inhibitor.
63. The composition of clause 37, wherein the secondary anti-hyperlipidemic or adjunctive therapeutic agent is an acyl-coenzyme A cholesterol acyltransferase (ACAT) inhibitor.
64. The composition of clause 63, wherein the acyl-coenzyme A cholesterol acyltransferase (ACAT) inhibitor is melinamide or probucol.
65. The composition of clause 57, wherein the secondary anti-hyperlipidemic or adjunctive therapeutic agent is a cholesterol absorption inhibitor.
66. The composition of clause 65, wherein the cholesterol absorption inhibitor is β sitosterol or ezetimibe.
67. The composition of clause 57, wherein the secondary anti-hyperlipidemic or adjunctive therapeutic agent is an anion exchange resin.
68. The composition of clause 67, wherein the anion exchange resin is cholestyramine, colestipol, colesevelam or dialkylaminoalkyl derivative.
69. The composition of clause 57, wherein the secondary anti-hyperlipidemic or adjunctive therapeutic agent is a fibrate.
70. The composition of clause 69, wherein the fibrate is clofibrate, bezafibrate, fenofibrate or gemfibrozil.
71. The composition of clause 57, wherein the secondary anti-hyperlipidemic or adjunctive therapeutic agent is is an herbal-derived product or extract.
72. The composition of clause 71, wherein the herbal-derived product or extract is obtained or selected from the group consisting of curcumin, gugulipid, garlic, vitamin E, soy, soluble fiber, fish oil, green tea, carntitine, chromium coenzyme Q10, vitamin C, betacarotene, grape seed extract, pantothine, red yeast rice, and royal jelly.
73. The composition of clause 57, wherein the administration of berberine is anti-hyperlipidemia effective to decrease total cholesterol in said subject to about 200mg/dL.
74. The composition of clause 57, wherein the administration of berberine is anti-hyperlipidemia effective to decrease total cholesterol in said subject to about 175 mg/dL.
75. The composition of clause 57, wherein the administration of berberine is anti-hyperlipidemia effective to decrease LDL levels in said subject to about 130 mg/dL.
76. The composition of clause 57, wherein the administration of said effective amount of'the berberine compound or berberine related or derivative compound of Formula I is anti-hyperlipidemia effective to decrease LDL levels in said subject by about 20mg/dL to about 50mg/dL.
77. The composition of clause 53, wherein the administration of said effective amount of the berberine compound or berberine related or derivative compound of Formula I is anti-hyperlipidemia effective to decrease triglycerides in said subject to about 150 mg/dL.
78. The composition of clause 57, wherein the administration of'said effective amount of the berberine compound or berberine related or derivative compound of Formula I is anti-hyperlipidemia effective to decrease triglycerides in said subject by about 20mg/dL to about 50 mg/dL.
79. The composition of clause 57, wherein the administration of said effective amount of the berberine compound or berberine related or derivative compound of Formula I is anti-hyperlipidemia effective to decrease hs-CRP in said subject to about 2.0 mg/L.
80. The composition of clause 57, wherein the administration of berberine is anti-hyperlipidemia effective to decrease hs-CRP in said subject by about 0 5mg/L to about 2.0 mg/L.
81. A method of modulating LDLR expression in a mammalian subject comprising administering to said subject an effective amount of a berberine compound or berberine related or derivative compound of Formula I, or a pharmaceutically- acceptable salt, isomer, enantiomer, solvate, hydrate, polymorph or prodrug thereof 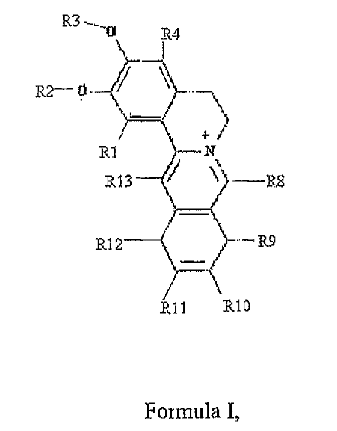 wherein each of R₁,R₂,R₃ R₄ R₈ R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl) alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups;
   and a secondary anti-hyperlipidemic agent or other adjunctive therapeutic agent useful in the treatment of a cardiovascular disease.
82. The method of clause 81, wherein R₁ is selected from methyl, ethyl, hydroxyl, or methoxy; R₂ is selected from H, methyl, ethyl, methane; R₃ is selected from H, methyl, ethyl, methene; R₄ is selected from methyl, ethyl, hydroxy, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1 dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2 dimethylbutyl, 3-methylpentyl, 1,2-dimethylbutyl, 1,3-dimethyl and 1-methyl-2ethylpropyl; R₉ is selected from methyl, ethyl, hydroxyl, C1, B1; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl,3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
83. The method of clause 81, wherein said mammalian subject is selected from a mammalian cell or cell culture, mammalian tissue or tissue explant, mammalian organ or organ explant, or a mammalian individual.
84. A composition for increasing LDLR expression in a mammalian subject comprising a LDLR increasing effective amount of berberine or a pharmaceutically-acceptable salt, isomer, enantiomer, solvate, hydrate, polymorph or prodrug thereof
85. A composition for increasing LDLR expression in a mammalian cell, tissue, organ, or individual comprising a LDLR effective amount of a berberine compound or berberine related or derivative compound of Formula I, or a pharmaceutically acceptable salt, isomer, enantiomer, solvate, hydrate, polymorph or produg thereof 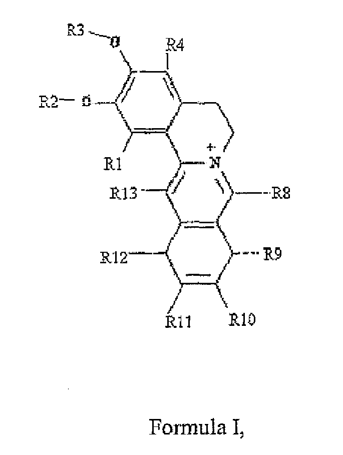 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groupsa berberine analog or a pharmaceutically-acceptable salt, isomer, enantiomer, solvate, hydrate, polymorph or prodrug thereof.
86. The composition of clause 85, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl,2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
87. The composition of clause 85, wherein the berberine compound or berberine related or derivative compound of Formula I is selected from the group consisting of berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetrahydroberberinium iodide, 6-protoberberine, 9-ethoxycarbonyl berberine, 3-N,N-dimethylcarbamoyl berberine and 12-bromo berberine, berberine azide, and berberine betaine.
88. A method for increasing LDLR stability in a mammalian cell, tissue, organ or individual comprising administering to a mammalian subject an effective amount of a berberine compound or berberine related or derivative compound of Formula I, or a pharmaceutically-acceptable salt, isomer, enantiomer, solvate, hydrate, polymorph or prodrug thereof 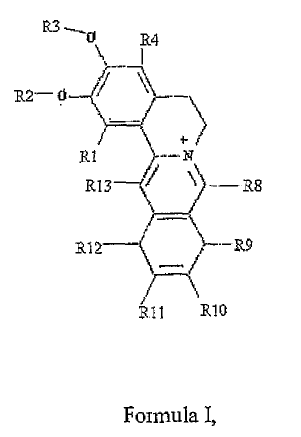 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groupsa berberine analog or a pharmaceutically-acceptable salt, isomer, enantiomer, solvate, hydrate, polymorph or prodrug thereof.
89. The method of clause 88, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
90. The method of clause 88, wherein the berberine compound or berberine related or derivative compound of Formula I is selected from the group consisting of berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetrahydroberberberinium iodide, 6-protoberberine, 9-ethoxycarbonyl berberine, 9-N,N-dimethylcarbamoyl berberine and 12-bromo berberine, berberine azide, and berberine betaine.
91. A composition for increasing LDLR stability in a mammalian cell, tissue, organ, or individual comprising an LDLR stabilizing amount of a berberine compound or berberine related or derivative compound of Formula I, or a pharmaceutically-acceptable salt, isomer, enantiomer, solvate, hydrate, polymorph or prodrug thereof 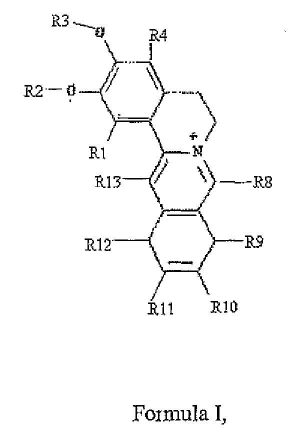 wherein each of R₁, R₂, R₃, R₄, R₈ R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groupsa berberine analog or a pharmaceutically-acceptable salt, isomer, enantiomer, solvate, hydrate, polymorph or prodrug thereof.
92. The composition of clause 91, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
93. The composition of clause 91, wherein the berberine compound or berberine related or derivative compound of Formula I is selected from the group consisting of berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetrahydroberberinium iodide, 6-protoberberine, 9-ethoxycarbonyl berberine, 9-N,N-dimethylcarbamoyl berberine and 12-bromo berberine, berberine, azide, and berberine betaine.
94. A method of modulating ERK activation in a mammalian subject selected from a mammalian cell, tissue, organ, or individual comprising administering to said subject an ERK activation modulatory effective amount of a berberine compound or berberine related or derivative compound of Formula I, or a pharmaceutically-acceptable salt, isomer, enantiomer, solvate, hydrate, polymorph or prodrug thereof 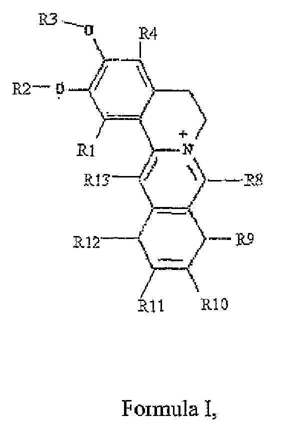 wherein each of R₁, R₂, R₃ R₄, R₈, R₉ R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groupsa berberine analog or a pharmaceutically-acceptable salt, isomer, enantiomer, solvate, hydrate, polymorph or prodrug thereof.
95. The method of clause 94, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2 ethylpropyl.
96. The method of clause 94, wherein the berberine compound of berberine related or derivative compound of Formula I is selected from the group consisting of berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetrahydroberberinium iodide, 6-protoberberine, 9-ethoxycarbonyl berberine, 9-N,N-dimethylcarbamoyl berberine and 12-bromo berberine, berberine azide, and berberine betaine.
97. A method of lowering cholesterol in a mammalian subject selected from a mammalian cell, tissue, organ, or individual comprising administering to said subject a cholesterol lowering effective amount of a berberine compound or berberine related or derivative compound of Formula I, or a pharmaceutically-acceptable salt, isomer, enantiomer, solvate, hydrate, polymorph or prodrug thereof 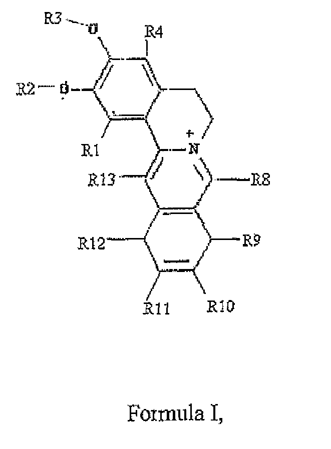 wherein each of R₁, R₂,R₃,R₄,R₈ R₉,R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groupsa berberine analog or a pharmaceutically-acceptable salt, isomer, enantiomer, solvate, hydrate, polymorph or prodrug thereof.
98. The method of clause 97, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or blanched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.
99. The method of clause 97, wherein the berberine compound or berberine related or derivative compound of Formula I is selected from the group consisting of berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetahydroberberinium iodide, 6-protoberberine, 9 ethoxycarbonyl berberine, 9-N,N-dimethylcarbamoyl berberine and 12-bromo berberine, berberine azide, and berberine betaine.
100. The method of clause 97, further comprising administering a second cholesterol lowering agent to said subject.
101. The method of clause 100, wherein the second cholesterol lowering agent is administered to said subject in a combined formulation with said berberine.
102. The method of clause 100, wherein the second cholesterol lowering agent is administered to said subject in a coordinate administration protocol, simultaneously with, prior to, or after, administration of said berberine to the subject.
103. The method of clause 100, wherein the second cholesterol lowering agent is selected from plasma HDL raising agents, cholesterol biosynthesis inhibitors, HMG-CoA reductase inhibitors, HMG-CoA synthase inhibitors, squalene epoxidase inhibitors, squalene synthetase inhibitors, acyl-coenzyme A cholesterol acyltransferase inhibitors, niacinamide, cholesterol absorption inhibitors, bile acid sequestrants, anion exchange resins, hormones, insulin, fibrates, vitamin B6, vitamin B12, vitamin B3, anti-oxidant vitamins, β blockers, angiotensin II receptor (AT₁) antagonists, angiotensin-converting enzyme inhibitors, renin inhibitors, platelet aggregation inhibitors, ion exchange resins, omega-3 oils, benfluorex, or cholesterol-uptake inhibitors.
104. The method of clause 100, wherein said cholesterol lowering effective amount comprises between about 20 mg to about 1500 mg of' said berberine compound or berberine related or derivative compound of Formula I per day.
105. The method of clause 100, wherein said cholesterol lowering effective amount comprises between about 25 mg to about 750 mg of said berberine compound or berberine related or derivative compound of Formula I per day.
106. The method of clause 100, wherein said cholesterol effective amount comprises between about 50 mg to about 500 mg of said berberine compound or berberine related of derivative compound of Formula I per day.
107. The method of clause 100, wherein said cholesterol lowering effective amount of said berberine compound or berberine related or derivative compound of Formula I is administered one, two, three, or four times per day.
108. The method of clause 100, wherein the administration of said effective amount of the berberine compound or berberine related or derivative compound of Formula I is effective to decrease total cholesterol in said subject to about 200mg/dL.
109. The method of clause 100, wherein the administration of said effective amount of the berberine, compound or berberine related or derivative compound of Formula I is effective to decrease total cholesterol in said subject to about 175 mg/dL.

## Claims

1. Use of an anti-hyperlipidemia effective amount of a berberine compound or berberine related or derivative compound of Formula I, or a pharmaceutically-acceptable salt, isomer, enantiomer, solvate, hydrate, polymorph or prodrug thereof, 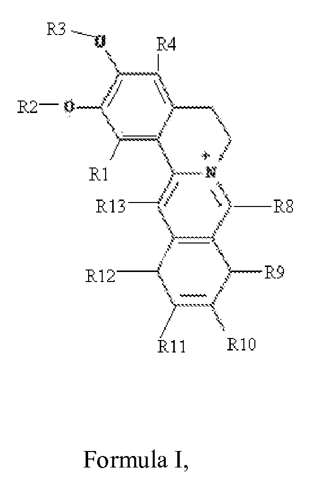 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups, for the manufacture of a medicament for controlling hyperlipidemia in a mammalian subject to reduce or prevent cardiovascular disease.

2. The use of claim 1, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2 ethylpropyl.

3. The use of claim 1, wherein the method is effective to reduce or prevent one or more symptoms, diseases, or conditions in said subject selected from atherosclerosis, coronary artery disease, angina pectoris, carotid artery disease, stroke, cerebral arteriosclerosis, high blood pressure, myocardial infarction, cerebral infarction, restenosis following balloon angioplasty, intermittent claudication, dyslipidemia post-prandial lipidemia, and xanthoma.

4. The use of claim 1, wherein the hyperlipidemia is associated with primary or secondary hyperlipidemia in said subject.

5. The use of claim 1, wherein the hyperlipidemia is associated with familial hyperchylomicronemia, familial hypercholesterolemia, familial combined hyperlipidemia, familial dysbetaliproteinemia, familial hypertriglyceridemia, familial defective apolipoprotein B-100, diabetes mellitus, hypothyroidism, uremia, nephrotic syndrome, acromegaly, obstructive liver disease, or dysproteinemia in said subject.

6. The use of claim 1, wherein the hyperlipidemia is associated with: prior or current use of oral contraceptives, glucocorticoids, or antihypertensives by said subject; or, adverse dietary habits of said subject.

7. The use of claim 1, wherein the administration of said effective amount of the berberine compound or berberine related or derivative compound of Formula I: is anti-hyperlipidemia effective to decrease total cholesterol in said subject to about 200mg/dL; or, is anti-hyperlipidemia effective to decrease total cholesterol in said subject to about 175 mg/dL; or, is anti-hyperlipidemia effective to decrease LDL levels in said subject to about 130 mg/dL; or, is anti-hyperlipidemia effective to decrease LDL levels in said subject by about 20mg/dL to about 50mg/Dl; or, is anti-hyperlipidemia effective to decrease triglycerides in said subject to about 150 mg/dL; or, is anti-hyperlipidemia effective to decrease triglycerides in said subject by about 20mg/dL to about 50 mg/dL; or, is anti-hyperlipidemia effective to decrease hs-CRP in said subject to about 2.0 mg/L; or, is anti-hyperlipidemia effective to decrease hs-CRP in said subject by about 0.5mg/L to about 2.0 mg/L.

8. Use of an effective amount of a berberine compound or berberine related or derivative compound of Formula I, or a pharmaceutically-acceptable salt, isomer, enantiomer, solvate, hydrate, polymorph or prodrug thereof 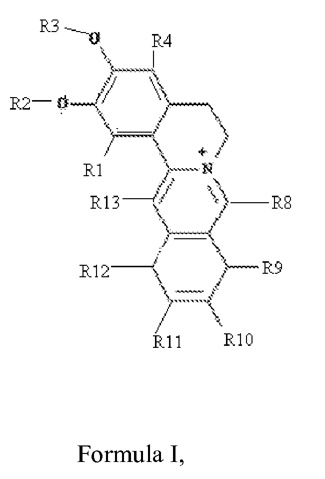 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groups; and a secondary anti-hyperlipidemic agent or other adjunctive therapeutic agent useful in the treatment of a cardiovascular disease, for the manufacture of a medicament for modulating LDLR expression in a mammalian subject.

9. The use of claim 8, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.

10. The use of claim 8, wherein said mammalian subject is selected from a mammalian cell or cell culture, mammalian tissue or tissue explant, mammalian organ or organ explant, or a mammalian individual.

11. Use of an ERK activation modulatory effective amount of a berberine compound or berberine related or derivative compound of Formula I, or a pharmaceutically-acceptable salt, isomer, enantiomer, solvate, hydrate, polymorph or prodrug thereof 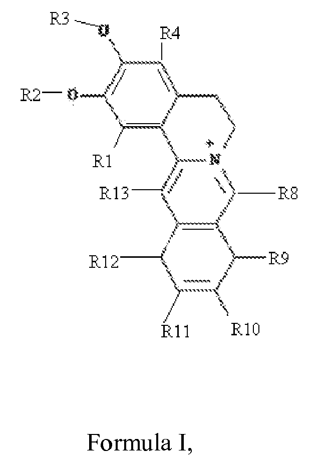 wherein each of R₁, R₂, R₃, R₄, R₈, R₉, R₁₀, R₁₁, R₁₂ and/or R₁₃ is, independently, collectively, or in any combination, selected from hydrogen, halogen, hydroxy, alkyl, alkoxy, nitro, amino, trifluoromethyl, cycloalkyl, (cycloalkyl)alkyl, alkanoyl, alkanoyloxy, aryl, aroyl, aralkyl, nitrile, dialkylamino, alkenyl, alkynyl, hydroxyalkyl, aminoalkyl, alkylaminoalkyl, dialkylaminoalkyl, haloalkyl, carboxyalkyl, alkoxyalkyl, carboxy, alkanoylamino, carbamoyl, carbamyl, carbonylamino, alkylsulfonylamino, and heterocyclo groupsa berberine analog or a pharmaceutically-acceptable salt, isomer, enantiomer, solvate, hydrate, polymorph or prodrug thereof, for the manufacture of a medicament for modulating ERK activation in a mammalian subject selected from a mammalian cell, tissue, organ, or individual.

12. The use of claim 11, wherein R₁ is selected from methyl, ethyl, hydroxyl, or methoxy; R₂ is selected from H, methyl, ethyl, methene; R₃ is selected from H, methyl, ethyl, methene; R₄ is selected from methyl, ethyl, hydroxyl, or methoxy; R₈ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl; R₉ is selected from methyl, ethyl, hydroxyl, Cl, Br; R₁₀ is selected from methyl, ethyl, hydroxyl, Cl, Br; R₁₁ is selected from methyl, ethyl, hydroxyl, Cl, Br; R₁₂ is selected from methyl, ethyl, hydroxyl, Cl, Br; and R₁₃ is selected from straight or branched (C1-C6) alkyl, including substitution selected from methyl, ethyl, n-propyl, 1-methylethyl, n-butyl, 1-methylpropyl, 2-methylpropyl, 1,1-dimethylethyl, n-pentyl, 2-methylbutyl, 1,1-dimethylpropyl, 2,2 dimethylpropyl, 3-methylbutyl, n-hexyl, 1-methylpentyl, 1,1-dimethylbutyl, 2,2-dimethylbutyl, 3-methylpentyl, 1,2-dimethylbutyl, 1,3-dimethyl and 1-methyl-2ethylpropyl.

13. The use of claim 11, wherein the berberine compound or berberine related or derivative compound of Formula I is selected from the group consisting of berberine sulfate, berberine hydrochloride, berberine chloride, palmatine chloride, oxyberberine, dihydroberberine, 8-cyanodihydroberberine, tetrahydroberberine N-oxide, tetrahydroberberine, N-methyltetrahydroberberinium iodide, 6-protoberberine, 9-ethoxycarbonyl berberine, 9-N,N-dimethylcarbamoyl berberine and 12-bromo berberine, berberine azide, and berberine betaine.
